# EUROPEAN PATENT SPECIFICATION

(11) **EP 4 103 611 B1**
(45) Date of publication and mention of the grant of the patent: **27.03.2024**
(21) Application number: 20710425.8
(22) Date of filing: 13.02.2020
(51) Int. Cl.: C07K 16/28, C07K 19/00

(54) **BISPECIFIC ANTIBODIES BINDING HVEM AND CD9**
BISPEZIFISCHE, AN HVEM UND CD9 BINDENDE ANTIKÖRPER
ANTICORPS BISPECIFIQUES SE LIANT A HVEM ET A CD9

(43) Date of publication of application: 21.12.2022
(73) Proprietor: UCB Biopharma SRL, 1070 Brussels (BE)
(72) Inventor: COOK, David Alan, Slough Berkshire SL1 3WE (GB); FINNEY, Helen Margaret, Slough Berkshire SL1 3WE (GB); RAPECKI, Stephen Edward, Slough Berkshire SL1 3WE (GB)
(74) Representative: UCB Intellectual Property
(86) International application number: PCT/EP2020/053773
(87) International publication number: WO 2021/160266

(56) References cited:
- WO-A1-2017/119811
- WO-A1-2017/174331
- WO-A2-2014/028560

## Description

### FIELD OF THE INVENTION

The present invention belongs to the field of multispecific antibodies binding at least HVEM and CD9, and their uses in the treatment of cancer and/or infectious diseases.

### BACKGROUND OF THE INVENTION

T-cells are key to a successful cell-mediated immune response necessary to eliminate cancer cells, bacteria and viruses. They recognise antigens displayed on the surface of tumour cells or antigens from bacteria and viruses replicating within the cells or from pathogens or pathogen products endocytosed from the extracellular fluid. T-cells have two major roles. They can become cytotoxic T-cells capable of destroying cells marked as foreign. Cytotoxic T-cells have a unique surface protein called CD8, thus they are often referred to as CD8+ T-cells. Alternatively, T-cells can become helper T-cells, which work to regulate and coordinate the immune system. Helper T-cells have a unique surface protein called CD4 and are thus often called CD4+ T-cells. Helper T-cells have several important roles in the immune system: 1) responding to activation by specific antigens by rapidly proliferating; 2) signaling B cells to produce antibodies; and 3) activating macrophages.

Cancer eludes the immune system by exploiting mechanisms developed to avoid auto-immunity. However, the immune system is programmed to avoid immune over-activation which could harm healthy tissue. T-cell activation is at the core of these mechanisms. Antigen specific T-cells normally able to fight disease can become functionally tolerant (exhausted) to infectious agents or tumour cells by over stimulation or exposure to suppressive molecules. Therefore, molecules that enhance the natural function of T-cells or overcome suppression of T-cells have great utility in the treatment or prevention of cancer and infectious disease.

Naïve T-cells are immature T-cells which are yet to have encountered antigen specific to their T-cell receptor, while memory T-cells have previously encountered antigen specific to their receptor and matured into effector cells, and as such can rapidly respond when the same antigen is presented. A key surface marker for identifying this change is the switch from the expression of CD45RA on naive T-cells to CD45RO on memory T-cells, and measuring the level of these cell surface proteins allows for the identification of the two populations. The immune system can identify tumour antigens, and mount a response, leading to the generation of memory T-cells.

However, the tumour microenvironment can dampen this response through multiple mechanisms, leading to tumour progression. The activation of these memory T-cells therefore offers great therapeutic value in reversing the immunosuppressive function of the tumour microenvironment. In recent years, immunotherapy has become an established treatment option for an increasing number of cancer patients, exemplified by the increased use of therapeutic antibody-based immune checkpoint inhibitors (CPI's). This has arisen from an increased immunological understanding of how cancer cells perturb immune cell activation by hijacking pathways normally involved in maintaining tolerance and skewing the balance between co-stimulation and co-inhibition (Chen and Mellman., Immunity. 39:1-105 (2013)). Amongst the pathways that have emerged as key regulators in this regard, include CTLA-4 and the PD-1/PD-L1 checkpoint molecules serving to down-regulate T cell and myeloid cell activation in the tumour microenvironment. Ipilimumab (anti-CTLA-4) was the first CPI to be approved in 2011 as a treatment for melanoma, closely followed by FDA approval of anti-PD1 directed antibodies, pembrolizumab and nivolumab in 2014 (Hargadon et al., International Immunopharmacol. 62:29-39 (2018)). There are still significant challenges in understanding differences in efficacy across patient groups, ranging from complete responses, to treatment relapse and even failure to respond, (Haslam and Prasad. JAMA Network Open.5:2e192535 (2019)).

Despite the promising anti-tumour efficacy of several monoclonal antibodies, many cancers are refractory to treatments with a single antibody. Combinations of two or more antibodies are currently being tested in patients to provide improved methods of treatment. Development of bispecific antibodies is also a strategy, see WO 2014/028560 disclosing bispecific antibodies binding CD3 and CD9 redirecting T-cell to target cells and WO 2017/174331 disclosing bispecific antibodies binding CD25 and an immune checkpoint for tumor depletion, HVEM being cited in the list of immune checkpoint. To date, these therapies rely on rational design of known mechanisms of action and are largely based on combining antigen-specificities known to be independently effective in the treatment of cancer, either as combination therapies or in a bispecific antibody format. This state of the art approach is a limiting factor in the development of new therapies as it relies on known therapies. Therefore, there is still the need to identify novel modulators of T-cell activation based on unbiased biology, which allows identification of novel target pairs capable of enhancing, T-cell activation and induction of T-cell proliferation for the treatment of cancer and infectious diseases.

### SUMMARY OF THE INVENTION

The present invention addresses the above-identified need by providing in a first aspect an antibody which comprises a first antigen-binding portion binding HVEM and a second antigen-binding portion binding CD9.

In one embodiment of this first aspect, each of the antigen-binding portions of the antibody which comprises a first antigen-binding portion binding HVEM and a second antigen-binding portion binding CD9 is a monoclonal antigen-binding portion.

In another embodiment, each of the antigen-binding portions is independently selected from a Fab, a Fab', a scFv or a VHH. In yet another aspect, the antigen-binding portions are the antigen-binding portions of an IgG.

In another embodiment, the antibody which comprises a first antigen-binding portion binding HVEM and a second antigen-binding portion binding CD9, is chimeric, human or humanised, and preferably the antibody is humanised.

In another embodiment, the antibody comprises a heavy chain constant region selected from an IgG1, an IgG2, IgG3 or an IgG4 isotype, or a variant thereof.

In another embodiment, the antibody further comprises at least an additional antigen-binding portion. The additional antigen-binding portion may be capable of increasing the half-life of the antibody. Preferably the additional antigen-binding portion binds albumin, more preferably human serum albumin.

In one embodiment, the second antigen binding portion binds CD9 in CD9 loop 2, wherein preferably the second antigen-binding portion binds within amino acids 112 to 195 of SEQ ID NO: 2.

In another embodiment, the first antigen-binding portion binding HVEM of the antibody according to the invention comprises a first heavy chain variable region and a first light chain variable region and the second antigen-binding portion binding CD9 comprises a second heavy chain variable region and a second light chain variable region and wherein:
a. The first heavy chain variable region comprises a CDR-H1 comprising SEQ ID NO: 3, a CDR-H2 comprising SEQ ID NO: 4 and a CDR-H3 comprising SEQ ID NO: 5; and
b. The first light chain variable region comprises a CDR-L1 comprising SEQ ID NO: 6, a CDR-L2 comprising SEQ ID NO: 7 and a CDR-L3 comprising SEQ ID NO: 8; and
c. The second heavy chain variable region comprises a CDR-H1 comprising SEQ ID NO: 9, a CDR-H2 comprising SEQ ID NO: 10 and a CDR-H3 comprising SEQ ID NO: 11; and
d. The second light chain variable region comprises a CDR-L1 comprising SEQ ID NO: 12, a CDR-L2 comprising SEQ ID NO: 13 and a CDR-L3 comprising SEQ ID NO: 14;
   or
e. The first heavy chain variable region comprises SEQ ID NO: 15 and the first light chain variable region comprises SEQ ID NO: 17; and the second heavy chain variable region comprises SEQ ID NO: 19 and second light chain variable region comprises SEQ ID NO: 21;
   or
f. The first heavy chain variable region is encoded by a nucleotide sequence comprising SEQ ID NO: 16 and the first light chain variable region is encoded by a nucleotide sequence comprising SEQ ID NO: 18; and the second heavy chain variable region is encoded by a nucleotide sequence comprising SEQ ID NO: 20 and second light chain variable region is encoded by a nucleotide sequence comprising SEQ ID NO: 22.

In a second aspect of the present invention there is provided a pharmaceutical composition comprising the antibody according to the first aspect of the invention and all its embodiments and one or more pharmaceutically acceptable excipients.

In a third aspect, the invention provides for the antibody according to the first aspect of the invention and all its embodiments or the pharmaceutical composition according to the second aspect of the invention and all its embodiments for use in therapy.

In one embodiment of this third aspect, the use is for the treatment of cancer and/or an infectious disease. In another embodiment, the antibody or the composition according to the invention and all its embodiments are for use in the treatment of cancer concomitantly or sequentially to one or more additional cancer therapies.

In a fourth aspect of the present invention, there is provided for a method for treating a subject afflicted with cancer and/or an infectious disease, comprising administering to the subject a pharmaceutically effective amount of the antibody according to the first aspect of the invention and all its embodiments or the pharmaceutical composition according to the second aspect of the invention and all its embodiments.

In one embodiment of this fourth aspect, the antibody or the composition are administered concomitantly or sequentially to one or more additional cancer therapies.

In a fifth aspect, the invention provides for the use of an antibody according to the first aspect of the invention and all its embodiments or the pharmaceutical composition according to the second aspect of the invention and all its embodiments in the manufacture of a medicament for treating cancer.

In one embodiment of this fifth aspect, the antibody or the composition are administered concomitantly or sequentially to one or more additional cancer therapies.

### BRIEF DESCRIPTION OF THE DRAWINGS

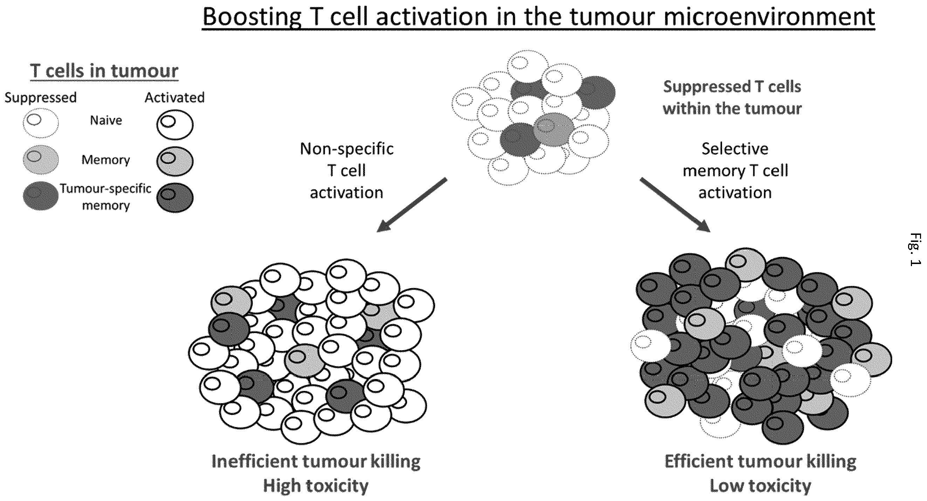

**Figure 1****.** Schematic representation of acquired immune resistance through selective memory T-cell activation versus non-specific T-cell activation.

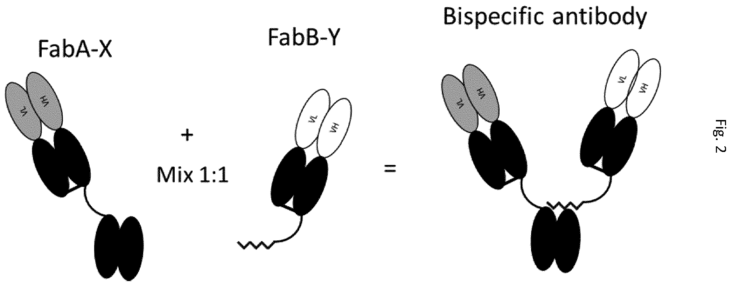

**Figure 2****.** General representation of a Fab-X and Fab-Y comprising antigen-binding portions and of the resulting bispecific antibody

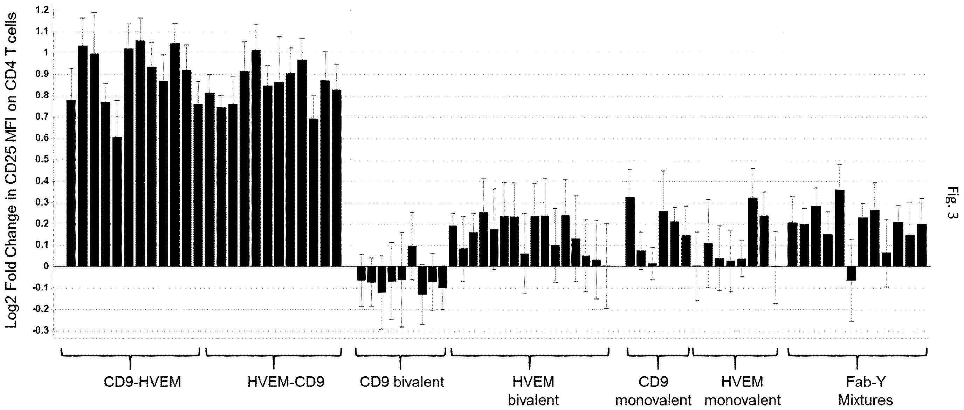

**Figure 3****.** Log2 fold change in the median fluorescence intensity (MFI) of CD25 expression on CD4+ T-cells in the presence of *Staphylococcus Aureus* Enterotoxin B (SEB) (1µg/mL) stimulation. PBMC cultures were treated with 1 µg/mL for 48 hours in the presence of either the HVEM-CD9 bispecific antibodies or control antibodies followed by flow cytometry for identifying the CD4+ T-cell population. Log2 fold changes were calculated for the MFI of CD25 levels in the treated samples relative to the SEB stimulated controls. N=4 donors, 2 technical replicates ± standard error of the mean (SEM).

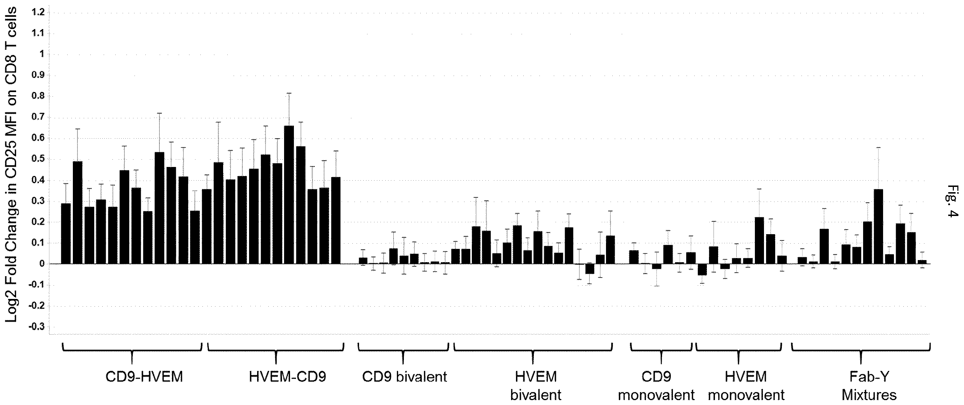

**Figure 4****.** Log2 fold change in the MFI of CD25 expression on CD8+ T-cells in the presence of SEB (1 µg/mL) stimulation. PBMC cultures were treated with *Staphylococcus Aureus* Enterotoxin B (SEB) at 1 µg/mL for 48 hours in the presence of either the HVEM-CD9 bispecific antibodies or control antibodies. The samples were then analysed by flow cytometry and the CD8+ T-cell population identified. Log2 fold changes were calculated for the MFI of CD25 levels in the treated samples relative to the SEB stimulated controls. N=4 donors, 2 technical replicates ± SEM.

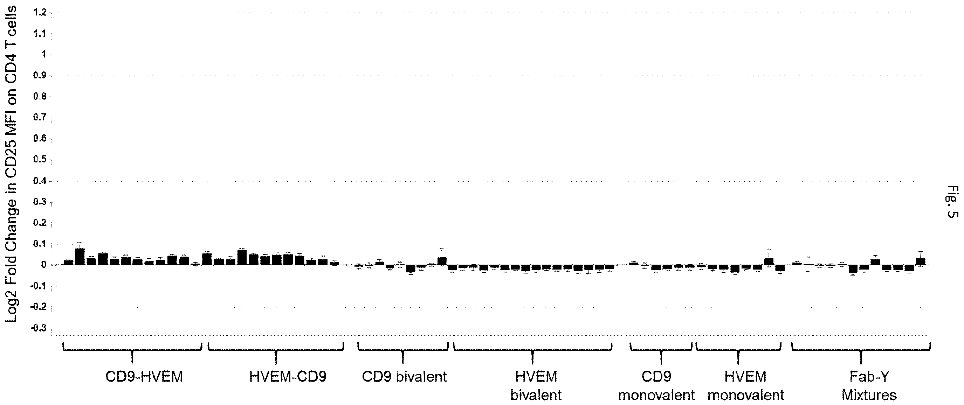

**Figure 5****.** Log2 fold change in the MFI of CD25 expression on CD4+ T-cells in the absence of any stimulation. PBMC were cultured for 48 hours in the presence of either the HVEM-CD9 bispecific antibodies or control antibodies. The samples were then analysed by flow cytometry and the CD4+ T-cell population identified. Log2 fold changes were calculated for the MFI of CD25 levels in the treated samples relative to the unstimulated controls. N=4 donors, 2 technical replicates± SEM.

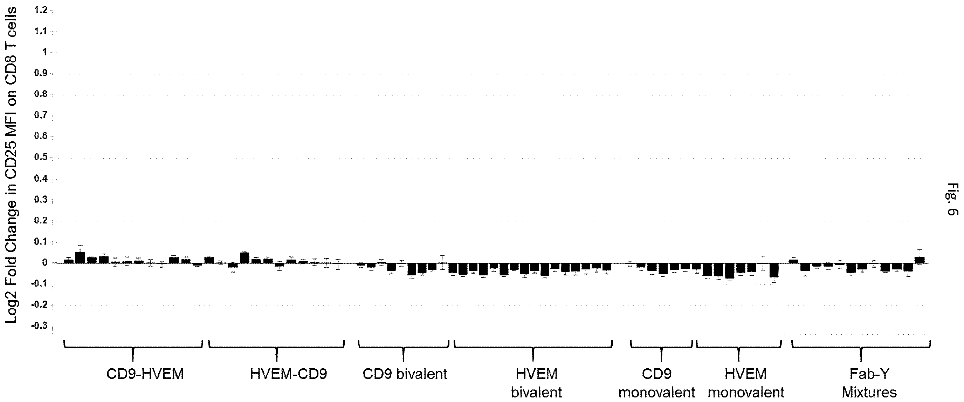

**Figure 6****.** Log2 fold change in the MFI of CD25 expression on CD8+ T-cells in the absence of any stimulation. PBMC were cultured for 48 hours in the presence of either the HVEM-CD9 bispecific antibodies or control antibodies. The samples were then analysed by flow cytometry and the CD8+ T-cell population identified. Log2 fold changes were calculated for the MFI of CD25 levels in the treated samples relative to the anti-CD3 stimulated controls. N=4 donors, 2 technical replicates ± SEM.

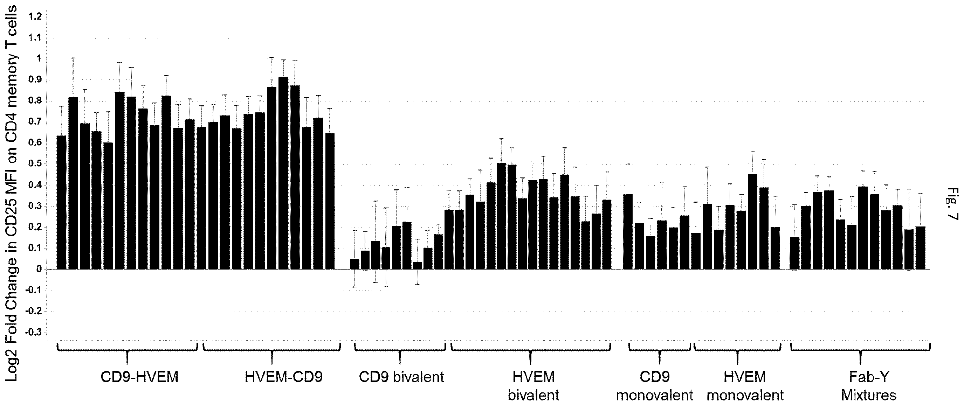

**Figure 7****.** Log2 fold change in the MFI of CD25 expression on CD4+ memory T-cells in the presence of SEB (1 µg/mL) stimulation. PBMC cultures were treated with *Staphylococcus Aureus* Enterotoxin B (SEB) at 1 µg/mL for 48 hours in the presence of either the HVEM-CD9 bispecific antibodies or control antibodies. The samples were then analysed by flow cytometry and the CD4+ T-cell population identified. Log2 fold changes were calculated for the MFI of CD25 levels in the treated samples relative to the SEB stimulated controls. N=4 donors, 2 technical replicates ± SEM.

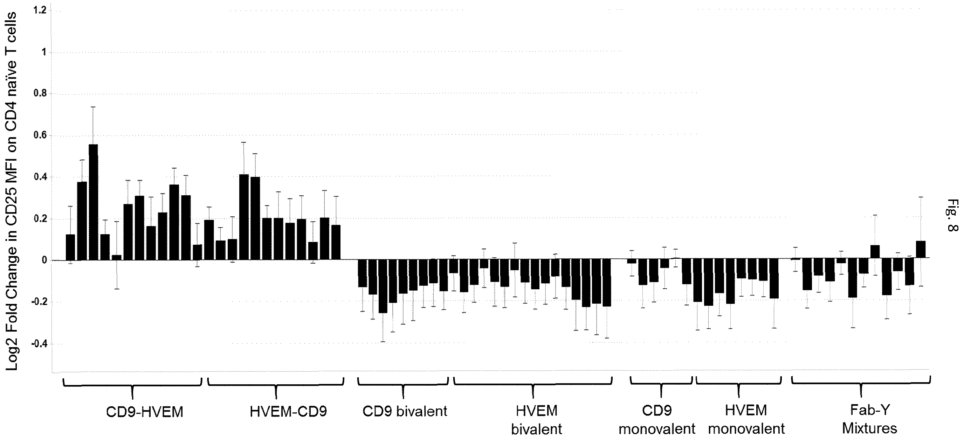

**Figure 8****.** Log2 fold change in the MFI of CD25 expression on CD4+ naive T-cells in the presence of SEB (1µg/mL) stimulation. PBMC cultures were treated with *Staphylococcus Aureus* Enterotoxin B (SEB) at 1µg/mL for 48 hours in the presence of either the HVEM-CD9 bispecific antibodies or control antibodies. The samples were then analysed by flow cytometry and the CD4+ T-cell population identified. Log2 fold changes were calculated for the MFI of CD25 levels in the treated samples relative to the SEB stimulated controls. N=4 donors, 2 technical replicates ± SEM.

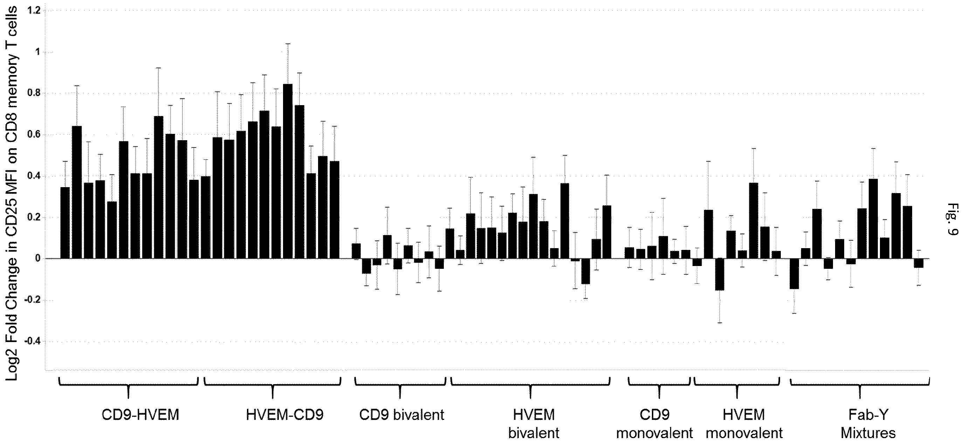

**Figure 9****.** Log2 fold change in the MFI of CD25 expression on CD8+ memory T-cells in the presence of SEB (1 µg/mL) stimulation. PBMC cultures were treated with *Staphylococcus Aureus* Enterotoxin B (SEB) at 1 µg/mL for 48 hours in the presence of either the HVEM-CD9 bispecific antibodies or control antibodies. The samples were then analysed by flow cytometry and the CD8+ T-cell population identified. Log2 fold changes were calculated for the MFI of CD25 levels in the treated samples relative to the SEB stimulated controls. N=4 donors, 2 technical replicates ± SEM.

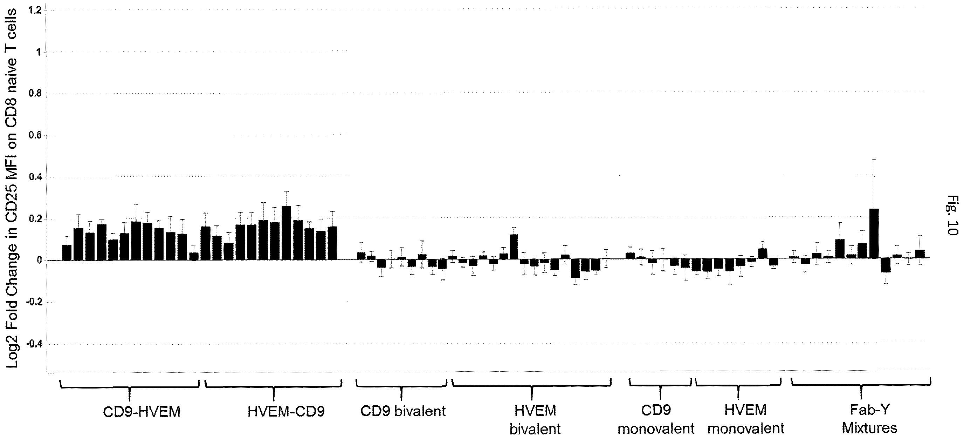

**Figure 10****.** Log2 fold change in the MFI of CD25 expression on CD8+ naive T-cells in the presence of SEB (1 µg/mL) stimulation. PBMC cultures were treated with *Staphylococcus Aureus* Enterotoxin B (SEB) at 1 µg/mL for 48 hours in the presence of either the HVEM-CD9 bispecific antibodies or control antibodies. The samples were then analysed by flow cytometry and the CD8+ T-cell population identified. Log2 fold changes were calculated for the MFI of CD25 levels in the treated samples relative to the SEB stimulated controls. N=4 donors, 2 technical replicates ± SEM.

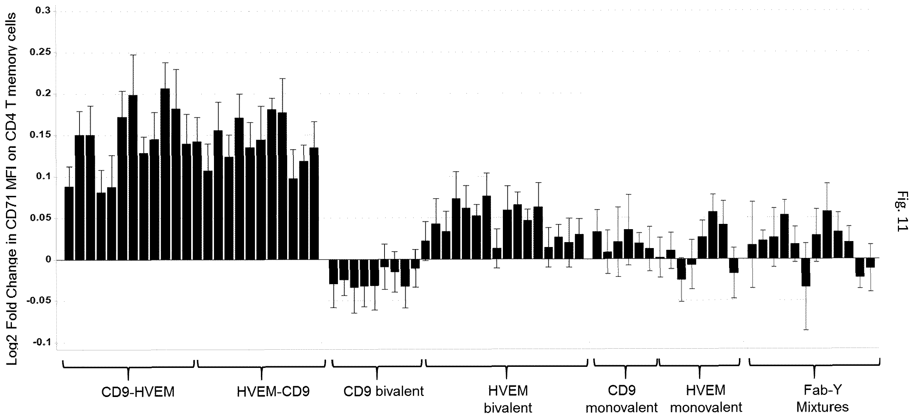

**Figure 11****.** Log2 fold change in the MFI of CD71 expression on CD4+ T memory cells in the presence of SEB (1 µg/mL) stimulation. PBMC cultures were treated with *Staphylococcus Aureus* Enterotoxin B (SEB) at 1 µg/mL for 48 hours in the presence of either the HVEM-CD9 bispecific antibodies or control antibodies. The samples were then analysed by flow cytometry and the CD4+ memory T-cell population identified. Log2 fold changes were calculated for the MFI of CD71 levels in the treated samples relative to the SEB stimulated controls. N=4 donors, 2 technical replicates ± SEM.

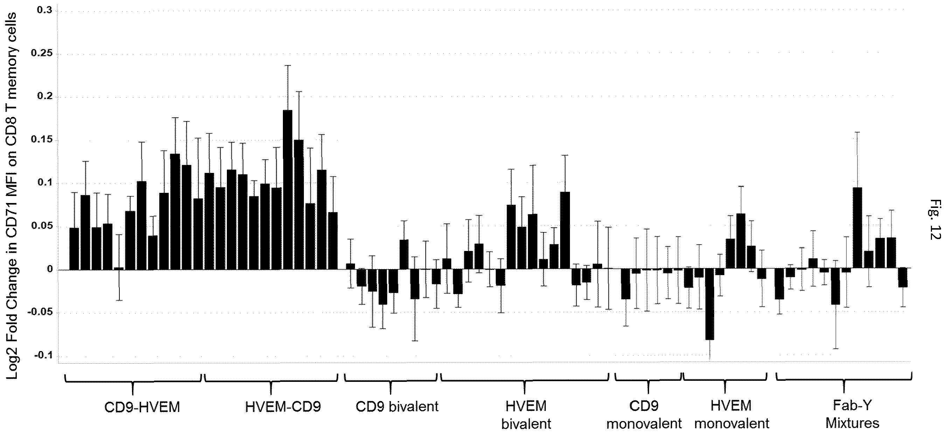

**Figure 12****.** Log2 fold change in the MFI of CD71 expression on CD8+ T memory cells in the presence of SEB (1 µg/mL) stimulation. PBMC cultures were treated with *Staphylococcus Aureus* Enterotoxin B (SEB) at 1 µg/mL for 48 hours in the presence of either the HVEM-CD9 bispecific antibodies or control antibodies. The samples were then analysed by flow cytometry and the CD8+ memory T-cell population identified. Log2 fold changes were calculated for the MFI of CD71 levels in the treated samples relative to the SEB stimulated controls. N=4 donors, 2 technical replicates ± SEM.

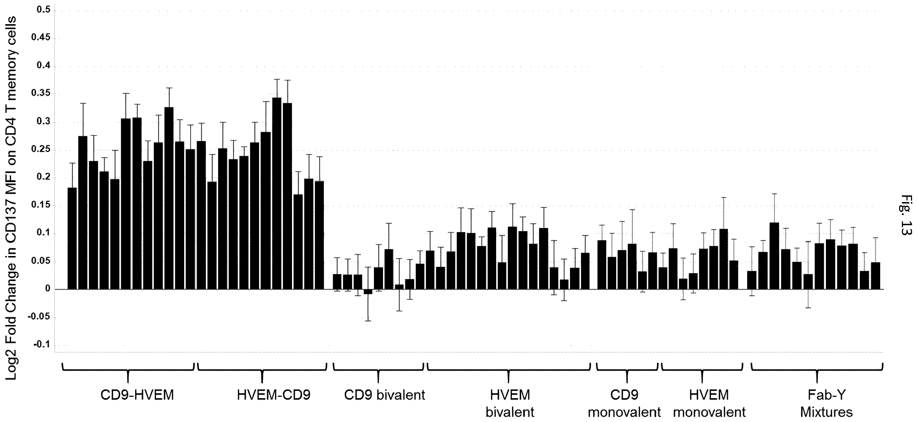

**Figure 13****.** Log2 fold change in the MFI of CD137 expression on CD4+ T memory cells in the presence of SEB (1µg/mL) stimulation. PBMC cultures were treated with *Staphylococcus Aureus* Enterotoxin B (SEB) at 1 µg/mL for 48 hours in the presence of either the HVEM-CD9 bispecific antibodies or control antibodies. The samples were then analysed by flow cytometry and the CD4+ memory T-cell population identified. Log2 fold changes were calculated for the MFI of CD137 levels in the treated samples relative to the SEB stimulated controls. N=4 donors, 2 technical replicates ± SEM.

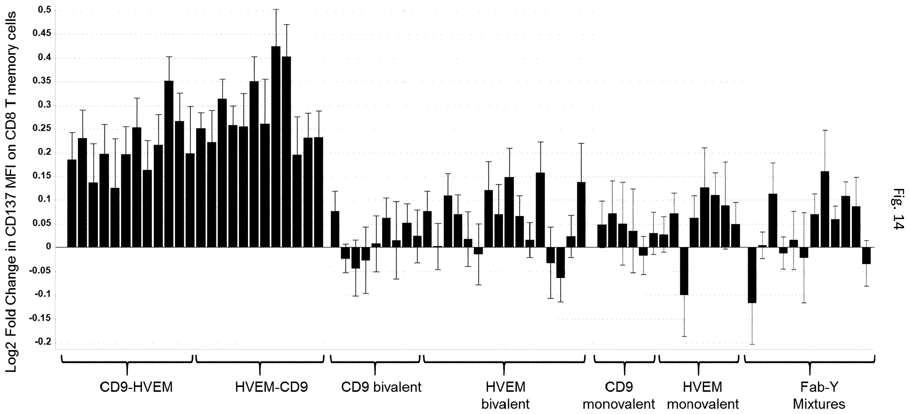

**Figure 14****.** Log2 fold change in the MFI of CD137 expression on CD8+ T memory cells in the presence of SEB (1 µg/mL) stimulation. PBMC cultures were treated with *Staphylococcus Aureus* Enterotoxin B (SEB) at 1 µg/mL for 48 hours in the presence of either the HVEM-CD9 bispecific antibodies or control antibodies. The samples were then analysed by flow cytometry and the CD8+ memory T-cell population identified. Log2 fold changes were calculated for the MFI of CD137 levels in the treated samples relative to the SEB stimulated controls. N=4 donors, 2 technical replicates ± SEM.

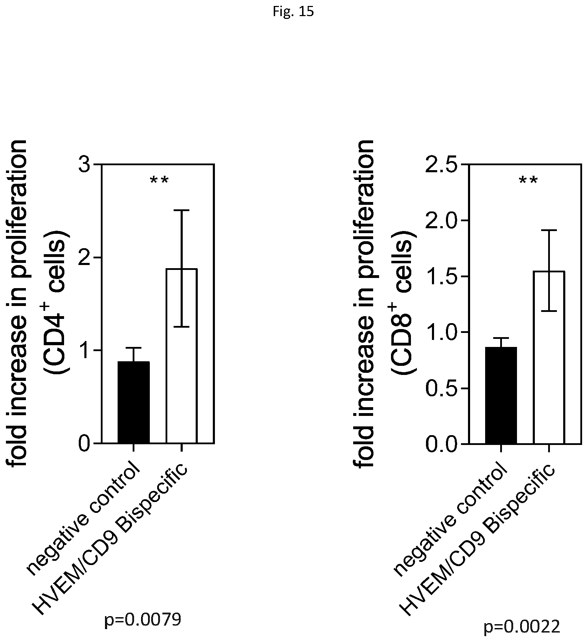

**Figure 15****.** Numbers of proliferating CD8+ and CD4+ T-cells in the presence of anti-CD3 (50 ng/mL; clone UCHT-1) stimulation. Human PBMC were labelled with CellTrace^{™} Violet (CTV) then incubated in triplicate for 4 days with anti-CD3 (clone UCHT-1) plus 100 nM of the HVEM-CD9 bispecific antibodies. T-cell proliferation was assessed by flow cytometry by gating on CD8+ and CD4+ populations and enumeration of CTV low cells. Results are presented as the mean ± SEM of 5 PBMC donors and statistically significant differences with a p value < 0.01 highlighted (Mann-Whitney test).

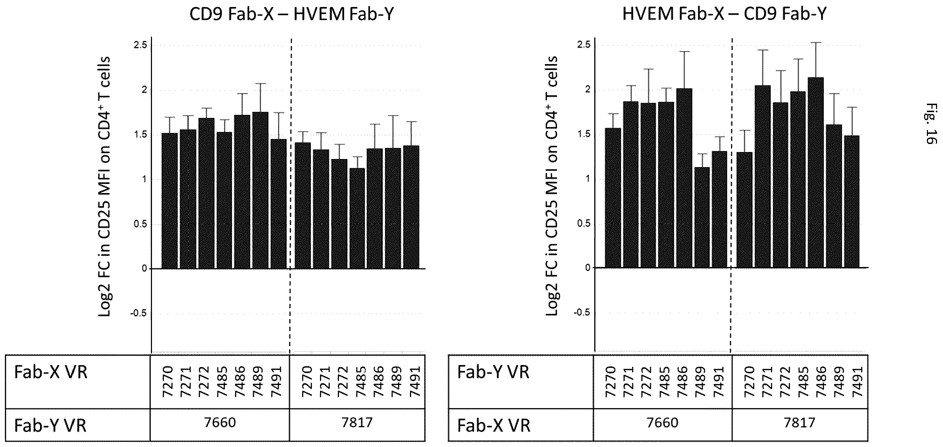

**Figure 16****.** Log₂ Fold Change in the MFI of CD25 expression on CD4⁺ T cells in the presence of SEB (1 µg/mL) stimulation. PBMC cultures were treated with *Staphylococcus aureus* Enterotoxin B (SEB) at 1 µg/mL for 48 hours in the presence of the HVEM-CD9 bispecific antibodies. The samples were then analysed by flow cytometry and the CD4⁺ T cell population identified. Log2 fold changes were calculated for the MFI of CD25 levels in the treated samples relative to the SEB stimulated controls. N=2 donors, 2 technical replicates. ± SEM.

### DETAILED DESCRIPTION OF THE INVENTION

The present invention will now be described with respect to particular non-limiting aspects and embodiments thereof and with reference to certain figures and examples.

Technical terms are used by their common sense unless indicated otherwise. If a specific meaning is conveyed to certain terms, definitions of terms will be given in the context of which the terms are used.

Where the term "comprising" is used in the present description and claims, it does not exclude other elements. For the purposes of the present disclosure, the term "consisting of" is considered to be a preferred embodiment of the term "comprising of".

Where an indefinite or definite article is used when referring to a singular noun, e.g. "a", "an" or "the", this includes a plural of that noun unless something else is specifically stated.

As used herein, the terms "treatment", "treating" and the like, refer to obtaining a desired pharmacologic and/or physiologic effect. The effect may be prophylactic in terms of completely or partially preventing a disease or symptom thereof and/or may be therapeutic in terms of a partial or complete cure for a disease and/or adverse effect attributable to the disease. Treatment thus covers any treatment of a disease in a mammal, particularly in a human, and includes: (a) preventing the disease from occurring in a subject, i.e. a human, which may be predisposed to the disease but has not yet been diagnosed as having it; (b) inhibiting the disease, i.e., arresting its development; and (c) relieving the disease, i.e., causing regression of the disease.

A "therapeutically effective amount" refers to the amount of antibody comprising the distinct antigen-binding portions binding HVEM and CD9 that, when administered to a mammal or other subject for treating a disease, is sufficient to affect such treatment for the disease.

The present invention provides for antibodies comprising a first antigen-binding portion binding HVEM and a second antigen-binding portion binding CD9. The first and the second antigen-binding portions are located in the same antibody, i.e. they are part of the same polypeptide chain and/or associate via one or more covalent and/or non-covalent associations (such as the screening format Fab-Kd-Fab described herein or the classic heavy and light chain association forming a full IgG antibody) or are covalently linked so as to form one single molecule (such as cross-linking two separately expressed polypeptide chains, optionally via specific cross-linking agents).

The herpes virus entry mediator (HVEM) is a member of the tumour necrosis factor receptor superfamily (TNFRSF), and it is also known as TNFRSF14 or CD270 (Uniprot accession reference Q92956). HVEM is a bidirectional switch regulating T-cell activation in a costimulatory or coinhibitory fashion, whose outcome depends on the binding partner. HVEM can act as both receptor and ligand, the binding of endogenous ligand LIGHT or agonist antibodies to HVEM delivers a costimulatory signal; whereas the binding of HVEM to BTLA (IgSF) or CD160 on EffectorT-cells delivers a coinhibitory signal. The sequence of human HVEM, including the signal peptide is shown as SEQ ID NO: 1 (Table 1).

CD9 and in particular human CD9 (Uniprot accession number P21926) was discovered as a human B-lymphocyte differentiation antigen and it has been found to be widely expressed on many non-hematopoietic tissues including various cancer. It is also known as Tetraspanin-29, motility-related protein-1, 5H9 antigen, cell growth-inhibiting gene 2 protein, leukocyte antigen MIC3 and MRP-1 (Rappa et al., Oncotarget 6:10, 7970-7991 (2015)). CD9 is a tetraspanin that is broadly expressed in a variety of solid tissues and on a multitude of hematopoietic cells (Nature reviews Cancer (9) 40-55 (2009)). CD9 involvement has been shown in the invasiveness and tumorigenicity of human breast cancer cells (Oncotargets, 6:10 (2015)), the suppression of cell motility and metastasis (J. Exp. Med 177:5 (1993)) and to have a role in T cells activation (J. Exp. Med 184:2 (1994)).

CD9 has also been shown to be present in exosomes (Asia-Pac J Clin Oncol, 2018; 1-9). Exosomes are cell derived nanovesicles with size of 30-120nm. The molecular composition of exosomes reflects their origin and include unique composition of tetraspanins. Exosomes are thoughts to constitute a potent mode of intercellular communication that is important in the immune response, cell-to-cell spread of infectious agents, and tumour progression.

The sequence of human CD9, including the signal peptide is shown as SEQ ID NO:2 (Table 1).

**Table 1**

| | |
|---|---|
| SEQ ID NO:1 Human HVEM | MEPPGDWGPPPWRSTPKTDVLRLVLYLTFLGAPCYAPALPSCKEDEY PVGSECCPKCSPGYRVKEACGELTGTVCEPCPPGTYIAHLNGLSKCLQ CQMCDPAMGLRASRNCSRTENAVCGCSPGHFCIVQDGDHCAACRAY ATSSPGQRVQKGGTESQDTLCQNCPPGTFSPNGTLEECQHQTKCSW LVTKAGAGTSSSHWVWWFLSGSLVIVIVCSTVGLIICVKRRKPRGDVVK VIVSVQRKRQEAEGEATVIEALQAPPDVTTVAVEETIPSFTGRSPNH MPVKGGTKCIKYLLFGFNFIFWLAGIAVLAIGLWLRFDSQTKSIFEQETN NNNSSFYTGVYILIGAGALMMLVGFLGCCGAVQESQCMLGLFFGFLLVI FAIEIAAAIWGYSHKDEVIKEVQEFYKDTYNKLKTKDEPQRETLKAIHYAL NCCGLAGGVEQFISDICPKKDVLETFTVKSCPDAIKEVFDNKFHIIGAVGI GIAVVMIFGMIFSMILCCAIRRNREMV |
| SEQ ID NO: 2 Human CD9 | MPVKGGTKCIKYLLFGFNFIFWLAGIAVLAIGLWLRFDSQTKSIFEQETN NNNSSFYTGVYILIGAGALMMLVGFLGCCGAVQESQCMLGLFFGFLLVI FAIEIAAAIWGYSHKDEVIKEVQEFYKDTYNKLKTKDEPQRETLKAIHYAL NCCGLAGGVEQFISDICPKKDVLETFTVKSCPDAIKEVFDNKFHIIGAVGI GIAVVMIFGMIFSMILCCAIRRNREMV |
| SEQ ID NO: 3 CDR-H1 VR7660 | GFSLSTYDMI |
| SEQ ID NO: 4 CDR-H2 VR7660 | VITKTTTTYYANWAKG |
| SEQ ID NO: 5 CDR-H3 VR7660 | DADDAVYYYFDI |
| SEQ ID NO: 6 CDR-L1 VR7660 | QASQSIGSGLA |
| SEQ ID NO: 7 CDR-L2 VR7660 | AASTLTS |
| SEQ ID NO: 8 CDR-L3 VR7660 | QEGVSRGNIDNA |
| SEQ ID NO: 9 CDR-H1 VR7272 | GFSLSSYAMG |
| SEQ ID NO: 10 CDR-H2 VR7272 | AIGSITATGYARWAKG |
| SEQ ID NO: 11 CDR-H3 VR7272 | EIYVGSAYAFDI |
| SEQ ID NO: 12 CDR-L1 VR7272 | QASQSISNYLA |
| SEQ ID NO: 13 CDR-L2 VR7272 | LASTLAS |
| SEQ ID NO: 14 CDR-L3 VR7272 | QQGYIDNVNKG |
| SEQ ID NO: 15 VH VR7660 | QSVEESGGRLVTPGTPLTLTCTVAGFSLSTYDMIWVRQAPGKGLEYIG VITKTTTTYYANWAKGRFTISKTSTTVDLKMTSPTTEDTATYFCARDAD DAVYYYFDIWGPGTLVTVSS |
| SEQ ID NO: 16 VH nucl. VR7660 | AAGCTTCGAAGCCACCATGGAGACTGGGCTGCGCTGGCTTCTCCTG GTCGCTGTGCTCAAAGGTGTCCAGTGTCAGTCGGTGGAGGAGTCC GGGGGTCGCCTGGTCACGCCTGGGACACCCCTGACACTCACCTGC ACGGTCGCTGGATTCTCCCTCAGTACCTACGACATGATCTGGGTCC GCCAGGCTCCAGGGAAGGGGCTGGAATACATCGGAGTCATTACTAA AACTACTACCACATACTACGCGAACTGGGCGAAAGGCCGATTCACC ATCTCCAAAACCTCGACCACGGTGGATCTGAAAATGACCAGTCCGA CAACCGAGGACACGGCCACCTATTTCTGTGCCAGAGATGCTGATGA TGCTGTTTATTACTACTTTGACATCTGGGGCCCAGGCACCCTGGTCA CCGTCTCGAGT |
| | |
| SEQ ID NO: 17 VL VR7660 | AYDMTQTPASAEVAVGGTVTINCQASQSIGSGLAWYQQKPGQRPKLLI YAASTLTSGVSSRFKGSGSGTEFTLTISGVECADAATYYCQEGVSRGNI DNAFGGGTEVVVK |
| SEQ ID NO: 18 VL nucl. VR7660 | AAGCTTCGAAGCCACCATGGACATGAGGGCCCCCACTCAGCTGCTG GGGCTCCTGCTGCTCTGGCTCCCAGGTGCCAGATGTGCCTATGATA TGACCCAGACTCCAGCCTCTGCGGAGGTAGCTGTGGGAGGCACAG TCACCATCAATTGCCAGGCCAGTCAGAGCATTGGCAGTGGTTTGGC CTGGTATCAGCAGAAACCAGGGCAGCGTCCCAAACTCCTGATCTAT GCTGCATCCACTCTGACATCTGGGGTCTCATCGCGGTTCAAAGGCA GTGGATCTGGGACAGAGTTCACTCTCACCATCAGCGGCGTGGAGTG TGCCGATGCTGCCACTTACTACTGTCAAGAGGGTGTTAGTCGTGGT AATATTGATAATGCCTTCGGCGGAGGGACCGAGGTGGTGGTCAAAC GTACG |
| SEQ ID NO: 19 VH VR7272 | QSLEESGGRLVTPGTPLTLTCTVSGFSLSSYAMGWVRQAPGKGLEWI GAIGSITATGYARWAKGRFSISKTSTTVDLKMTSPTTEDTATYFCAREIY VGSAYAFDIWGPGTLVTVSS |
| SEQ ID NO: 20 VH nucl. VR7272 | AAGCTTCGAAGCCACCATGGAGACTGGGCTGCGCTGGCTTCTCCTG GTCGCTGTGCTCAAAGGTGTCCAGTGTCAGTCGCTGGAGGAGTCC GGGGGTCGCCTGGTCACGCCTGGGACACCCCTGACACTCACCTGC ACAGTCTCTGGATTCTCCCTCAGTAGCTATGCAATGGGCTGGGTCC GCCAGGCTCCAGGGAAGGGGCTGGAGTGGATCGGAGCCATTGGTA GTATTACTGCCACTGGCTACGCGCGCTGGGCAAAAGGCCGATTCAG CATCTCCAAGACCTCGACCACGGTGGATCTGAAAATGACCAGTCCG ACAACCGAGGACACGGCCACCTATTTCTGTGCCAGAGAGATTTATG TTGGGTCTGCTTATGCCTTTGACATCTGGGGCCCAGGCACCCTGGT CACCGTCTCGAGT |
| SEQ ID NO: 21 VL VR7272 | AYDMTQTPASVEVAVGDTVTIKCQASQSISNYLAWYQQKPGQPPKLLIY LASTLASGVPSRFKGSGSGTEFTLTISDLECADAATYYCQQGYIDNVNK GFGGGTEVVVK |
| SEQ ID NO: 22 VL nucl. VR7272 | AAGCTTCGAAGCCACCATGGACACGAGGGCCCCCACTCAGCTGCT GGGGCTCCTGCTGCTCTGGCTCCCAGGTGCCAGATGTGCCTATGAT ATGACCCAGACTCCAGCCTCTGTGGAGGTAGCTGTGGGAGACACTG TCACCATCAAGTGTCAGGCCAGTCAGAGCATTAGTAACTACTTAGCC TGGTATCAGCAGAAACCAGGGCAGCCTCCCAAGCTCCTGATCTATC TGGCATCTACTCTGGCATCTGGGGTCCCATCGCGGTTCAAAGGCAG TGGATCTGGGACAGAGTTCACTCTCACCATCAGCGACCTGGAGTGT GCCGATGCTGCCACTTACTATTGTCAACAGGGTTATATTGATAATGT TAATAAAGGTTTCGGCGGAGGGACCGAGGTGGTGGTCAAACGTAC G |

Within the present invention, unless recited otherwise, human HVEM and CD9 are always intended to be included in the term "HVEM" and CD9". However, unless "human HVEM" and/or "human CD9" are explicitly used, the terms "HVEM" and/or "CD9" include the same targets in other species, especially non-primate (e.g. rodents) and non-human primate (such as cynomolgus monkey) species.

The present invention therefore provides for an antibody comprising a first antigen-binding portion binding human HVEM and a second antigen-binding portion binding human CD9. The first and the second antigen-binding portions are located on the same antibody, i.e. they are part of the same polypeptide chain, they associate via one or more non-covalent and/or covalent associations or are linked so as to form one single molecule.

The present invention also provides for an antibody comprising a first antigen-binding portion binding an extracellular domain region of human HVEM and a second antigen-binding portion binding an extracellular domain region of human CD9.

In particular, there is provided an antibody comprising a first antigen-binding portion binding human HVEM as defined in SEQ ID NO: 1 or from amino acid 39 to 283 of SEQ ID NO: 1 or from amino acid 39 to 202 of SEQ ID NO: 1 and a second antigen-binding portion binding human CD9 as defined in SEQ ID NO: 2 or from amino acid 2 to 228 of SEQ ID NO: 2, alternatively from amino acid 34 to 55 of SEQ ID NO: 2 or alternatively from amino acid 112-195. The first and the second antigen-binding portions are located on the same antibody, i.e. they are part of the same polypeptide chain, they associate via one or more non-covalent and/or covalent associations or are linked so as to form one single molecule.

The monoclonal antibody of the present invention, upon binding of HVEM and CD9, stimulates T-cell activation, i.e. further activates T-cells and enhances induction of T-cell proliferation, and in particular, the monoclonal antibody comprising a first antigen-binding portion binding HVEM and a second antigen-binding portion binding CD9 further activates T-cells and enhances induction of T-cell proliferation in the presence of SEB stimulation. More specifically, the monoclonal antibody comprising a first antigen-binding portion binding HVEM and a second antigen-binding portion binding CD9 further activates T-cells and enhances induction of T-cell proliferation in the presence of SEB stimulation but it does not activate unstimulated T-cells. More specifically, the T-cell is at least a CD4+ T-cell or at least a CD8+ T-cell or a mixture thereof, more preferably a memory CD4+ T-cell or a memory CD8+ T-cell.

The term "activate" (and grammatical variations thereof) as used herein, at least includes the upregulation of specific T-cell markers, i.e. increased transcription and/or translation of these markers and/or trafficking of these newly transcribed/translated markers or of any marker already expressed to the cell membrane, and the induction of proliferation.

Hence, the present invention provides for a monoclonal antibody comprising a first antigen-binding portion binding HVEM and a second antigen-binding portion binding CD9 capable of activating T-cells in the presence of an anti-SEB stimulation wherein the further activation of T-cell is measured as an upregulation of T-cell markers and the enhancement of T-cell proliferation.

For maintaining the ability of the immune system to recognise and mount a response to the almost infinite number of possible foreign peptide antigens, a large repertoire of naive T-cells is required. When a foreign antigen is encountered - for example as presented on virus infected cells or mutated tumour cells - T-cells of that specificity are selectively expanded to allow killing of the virus or tumour. As part of this selective expansion a number of memory T-cells are formed and retained at increased frequency to allow a rapid response should the same threat be re-encountered. This expansion and maintenance of memory T-cells is the basis of acquired immune resistance.

Stimulation of the cytotoxic activity of T-cells to promote tumour or virus-infected cell killing is a desirable therapeutic approach - but if non-specific can be catastrophic due to massive cytokine release and indiscriminate cell killing (Figure 1). Agents designed to selectively stimulate these memory T-cells will promote a more specific, localised and effective immune response without overt immune activation and toxicity.

Hence, the present invention provides for a monoclonal antibody comprising a first antigen-binding portion binding HVEM and a second antigen-binding portion binding CD9 capable of further activating memory T-cells, wherein the further activation of T-cell results in an upregulation of T-cell markers and the enhancement of memory T-cell proliferation.

Specific markers of T-cell activation and proliferation include but are not limited to the upregulation of CD25, CD137 and CD71.

In one preferred embodiment of the present invention, the monoclonal antibody comprising a first antigen-binding portion binding HVEM and a second antigen-binding portion binding CD9 is capable of activating T-cells in the presence of SEB stimulation wherein activating T-cell results in an upregulation of CD137, CD71 and CD25.

The term "antibody" as used herein includes whole immunoglobulin molecules and antigen-binding portions of immunoglobulin molecules associated via non-covalent and/or covalent associations or linked together, optionally via a linker.

In one embodiment, the antigen-binding portions binding HVEM and CD9 are the antigen-binding portions of an IgG, wherein one arm binds HVEM and the other arm binds CD9.

In another embodiment, the antigen-biding portions comprised in the antibody are functionally active fragments or derivatives of a whole immunoglobulin and may be, but are not limited to, VH, VL, VHH, Fv, scFv fragment (including dsscFv), Fab fragments, modified Fab fragments, Fab' fragments, F(ab')₂ fragments, Fv and epitope-binding fragments of any of the above.

Other antibody fragments include those described in WO2005003169, WO2005003170, WO2005003171, WO2009040562 and WO2010035012. Functionally active fragments or derivative of a whole immunoglobulin and methods of producing them are well known in the art, see for example Verma et al., 1998, Journal of Immunological Methods, 216, 165-181; Adair and Lawson, 2005. Therapeutic antibodies. Drug Design Reviews - Online 2(3):209-217.

In one embodiment of the invention each of the antigen-binding portions is independently selected from a Fab, a Fab', a scFv or a VHH. In one embodiment, the antigen-binding portion binding HVEM is a Fab whilst the antigen-binding portion binding CD9 is a scFv. In another embodiment, the antigen-binding portion binding CD9 is a Fab whilst the antigen-binding portion binding HVEM is a scFv. In another embodiment, both antigen-binding portions are a Fab or scFv.

In one preferred embodiment, the antibody is monoclonal, which means that the antigen-binding portions comprised therein are all monoclonal. Therefore, in one preferred embodiment of the present invention, there is provided a monoclonal antibody comprising a first antigen-binding portion binding HVEM and a second antigen-binding portion binding CD9. Preferably, this antibody is capable of further activating T-cells and/or enhancing induction of T-cell proliferation in the presence of SEB stimulation wherein activating T-cells results in an upregulation of CD71, CD137 and CD25.

Monoclonal antibodies may be prepared by any method known in the art such as the hybridoma technique (Kohler & Milstein, 1975, Nature, 256:495-497), the trioma technique, the human B-cell hybridoma technique (Kozbor et al., 1983, Immunology Today, 4:72) and the EBV- hybridoma technique (Cole et al., Monoclonal Antibodies and Cancer Therapy, pp 77-96, Alan R Liss, Inc., 1985).

Antibodies for use in the invention may also be generated using single lymphocyte antibody methods by cloning and expressing immunoglobulin variable region cDNAs generated from single lymphocytes selected for the production of specific antibodies by for example the methods described by Babcook, J. et al, 1996, Proc. Natl. Acad. Sci. USA 93(15):7843-78481; WO92/02551; WO2004/051268 and WO2004/106377.

The antibodies of the present invention can also be generated using various phage display methods known in the art and include those disclosed by Brinkman et al. (in J. Immunol. Methods, 1995, 182: 41-50), Ames et al. (J. Immunol. Methods, 1995, 184: 177-186), Kettleborough et al. (Eur. J. Immunol. 1994, 24:952-958), Persic et al. (Gene, 1997 187 9-18), Burton et al. (Advances in Immunology, 1994, 57: 191-280) and WO 90/02809; WO 91/10737; WO 92/01047; WO 92/18619; WO 93/11236; WO 95/15982; WO 95/20401; and U.S. Pat. Nos. 5,698,426; 5,223,409; 5,403,484; 5,580,717; 5,427,908; 5,750,753; 5,821,047; 5,571,698; 5,427,908; 5,516,637; 5,780,225; 5,658,727; 5,733,743 and 5,969,108. When the antigen-binding portions comprised in the antibody are functionally active fragments or derivatives of a whole immunoglobulin such as single chain antibodies, they may be made such as those described in U.S. Pat. No. 4,946,778 which can also be adapted to produce single chain antibodies binding to HVEM and CD9. Transgenic mice, or other organisms, including other mammals, may be used to express antibodies, including those within the scope of the invention.

The antibody of the present invention may be chimeric, human or humanised.

Chimeric antibodies are those antibodies encoded by immunoglobulin genes that have been genetically engineered so that the light and heavy chain genes are composed of immunoglobulin gene segments belonging to different species.

Humanized, antibodies are antibody molecules from non-human species having one or more complementarity determining regions (CDRs) from the non-human species and a framework region from a human immunoglobulin molecule (see, e.g. US5,585,089; WO91/09967). Preferably the antibody of the present invention is humanised. In one embodiment of the present invention, there is provided an antibody, preferably a monoclonal antibody, comprising a first antigen-binding portion binding HVEM and a second antigen-binding portion binding CD9, wherein the antibody is humanised. More preferably this antibody is capable of activating T-cells and/or enhancing induction of T-cell proliferation in the presence of SEB stimulation wherein activating T-cell results in an upregulation of CD137, CD71 and CD25.

In humanised antibodies, the heavy and/or light chain contains one or more CDRs (including, if desired, one or more modified CDRs) from a donor antibody (e.g. a murine monoclonal antibody) grafted into a heavy and/or light chain variable region framework of an acceptor antibody (e.g. a human antibody). For a review, see Vaughan et al, Nature Biotechnology, 16, 535-539, 1998. In one embodiment, rather than the entire CDR being transferred, only one or more of the specificity determining residues from any one of the CDRs described herein above are transferred to the human antibody framework (see, for example, Kashmiri et al., 2005, Methods, 36, 25-34). When the CDRs or specificity determining residues are grafted, any appropriate acceptor variable region framework sequence may be used having regard to the class/type of the donor antibody from which the CDRs are derived, including mouse, primate and human framework regions. Preferably, the humanised antibody according to the invention comprises a variable domain comprising human acceptor framework regions as well as one or more of the CDRs or specificity determining residues described above. Thus, provided in one embodiment is a humanised monoclonal antibody comprising an antigen-binding portion binding HVEM and an antigen-binding portion binding CD9, wherein each antigen-binding portion comprises a variable domain comprising human acceptor framework regions and non-human donor CDRs.

Examples of human frameworks which can be used in the invention are KOL, NEWM, REI, EU, TUR, TEI, LAY and POM (Kabat et al, supra). For example, KOL and NEWM can be used for the heavy chain, REI can be used for the light chain and EU, LAY and POM can be used for both the heavy chain and the light chain. Alternatively, human germline sequences may be used; these are available at, for example: http://vbase.mrc-cpe.cam.ac.uk/. In a CDR-grafted antibody of the invention, the acceptor heavy and light chains do not necessarily need to be derived from the same antibody and may, if desired, comprise composite chains having framework regions derived from different chains.

Fully human antibodies are those antibodies in which the variable regions and the constant regions (where present) of both the heavy and the light chains are all of human origin, or substantially identical to sequences of human origin, not necessarily from the same antibody. Examples of fully human antibodies may include antibodies produced for example by the phage display methods described above and antibodies produced by mice in which the murine immunoglobulin variable and constant region genes have been replaced by their human counterparts, e.g., as described in general terms in EP0546073, U.S. Pat. No. 5,545,806, U.S. Pat. No. 5,569,825, U.S. Pat. No. 5,625,126, U.S. Pat. No. 5,633,425, U.S. Pat. No. 5,661,016, U.S. Pat. No. 5,770,429, EP 0438474 and EP0463151.

Furthermore, the antibody of the invention may comprise a heavy chain constant region selected from an IgG1, an IgG2, an IgG3 or an IgG4 isotype, or a variant thereof. The constant region domains of the antibody of the invention, if present, may be selected having regard to the proposed function of the antibody, and in particular the effector functions which may be required. For example, the human IgG constant region domains of the lgG1 and IgG3 isotypes may be used when the antibody effector functions are required. Alternatively, IgG2 and IgG4 isotypes may be used when the antibody effector functions are not required. For example, IgG4 molecules in which the serine at position 241 has been changed to proline as described in Angal et al., Molecular Immunology, 1993, 30 (1), 105-108, may be used. Particularly preferred is the IgG4 constant domain that comprises this change.

It should also be appreciated that antigen-binding portions comprised in the antibody of the invention such as the functionally-active fragments or derivatives of whole immunoglobulin fragments described above, may be incorporated into other antibody formats than being the antigen-binding portions of the classic IgG format. Alternative format to the classic IgG may include those known in the art and those described herein, such as DVD-Igs, FabFvs for example as disclosed in WO2009/040562 and WO2010/035012, diabodies, triabodies, tetrabodies etc. Other examples include a diabody, triabody, tetrabody, bibodies and tribodies (see for example Holliger and Hudson, 2005, Nature Biotech 23(9): 1 126-1136; Schoonjans et al. 2001 , Biomolecular Engineering, 17 (6), 193-202); tandem scFv, tandem scFv-Fc, FabFv, Fab'Fv, FabdsFv, Fab-scFv, Fab'-scFv, diFab, diFab', scdiabody, scdiabody-Fc, ScFv-Fc-scFv, scdiabody-CH3, IgG-scFv, scFv-IgG, V-IgG, IgG-V, DVD-Ig, DuoBody, Fab-Fv-Fv, Fab-Fv-Fc and Fab-dsFv-PEG fragments described in WO2009040562, WO2010035012, WO2011/08609, WO2011/030107 and WO201 1/061492, respectively.

Furthermore, the antibody of the invention may comprise along with the antigen-binding portions binding HVEM and CD9, also at least an additional antigen-binding portion. Therefore, in one embodiment, there is provided an antibody, preferably a monoclonal antibody, comprising a first antigen-binding portion binding HVEM and a second antigen-binding portion binding CD9, wherein the antibody is humanised and wherein the antibody further comprises an additional antigen-binding portion. More preferably this antibody is capable of activating T-cells and/or enhancing induction of T-cell proliferation. The stimulation may be SEB stimulation wherein activating T-cells may result in an upregulation of CD137, CD71 and CD25.

In one embodiment, the additional antigen-binding portion is capable of increasing, i.e. extending, the half-life of the antibody. Preferably, the additional antigen-binding portion binds albumin, more preferably human serum albumin.

In one preferred embodiment, the antibody comprises a first antigen-binding portion binding the HVEM and a second antigen-binding portion binding an extracellular domain region of human CD9, wherein the extracellular domain region of CD9 is preferably loop 2 of CD9, and wherein more preferably the second antigen-binding portion binds within amino acids 112 to 195 of SEQ ID NO: 2, wherein the first and the second antigen-binding portions are located on the same antibody, i.e. they are part of the same polypeptide chain, associate via one or more non-covalent and/or covalent associations or linked so as to form one single molecule.

In another embodiment, the first antigen-binding portion binding HVEM of the antibody of the present invention comprises a first heavy chain variable region and a first light chain variable region and the second antigen-binding portion binding CD9 comprises a second heavy chain variable region and a second light chain variable region and wherein:
a. The first heavy chain variable region comprises a CDR-H1 comprising SEQ ID NO: 3, a CDR-H2 comprising SEQ ID NO: 4 and a CDR-H3 comprising SEQ ID NO: 5; and
b. The first light chain variable region comprises a CDR-L1 comprising SEQ ID NO: 6, a CDR-L2 comprising SEQ ID NO: 7 and a CDR-L3 comprising SEQ ID NO: 8; and
c. The second heavy chain variable region comprises a CDR-H1 comprising SEQ ID NO: 9, a CDR-H2 comprising SEQ ID NO: 10 and a CDR-H3 comprising SEQ ID NO: 11; and
d. The second light chain variable region comprises a CDR-L1 comprising SEQ ID NO: 12, a CDR-L2 comprising SEQ ID NO: 13 and a CDR-L3 comprising SEQ ID NO: 14;
   or
e. The first heavy chain variable region comprises SEQ ID NO: 15 and the first light chain variable region comprises SEQ ID NO: 17; and the second heavy chain variable region comprises SEQ ID NO: 19 and second light chain variable region comprises SEQ ID NO: 21;
   or
f. The first heavy chain variable region is encoded by a nucleotide sequence comprising SEQ ID NO: 16 and the first light chain variable region is encoded by a nucleotide sequence comprising SEQ ID NO: 18; and the second heavy chain variable region is encoded by a nucleotide sequence comprising SEQ ID NO: 20 and second light chain variable region is encoded by a nucleotide sequence comprising SEQ ID NO: 22.

In one embodiment, the antibody according to the present invention is prepared according to the disclosure of WO2015/181282, WO2016/009030, WO2016/009029, WO2017/093402, WO2017/093404 and WO2017/093406. More specifically, the antibody is made by the heterodimerization of a Fab-X and a Fab-Y.

Fab-X comprises a Fab fragment which comprises the first antigen-binding portion binding HVEM which comprises a first heavy chain variable region and a first light chain variable region wherein the first heavy chain variable region comprises a CDR-H1 comprising SEQ ID NO: 3, a CDR-H2 comprising SEQ ID NO: 4 and a CDR-H3 comprising SEQ ID NO: 5; and the first light chain variable region comprises a CDR-L1 comprising SEQ ID NO: 6, a CDR-L2 comprising SEQ ID NO: 7 and a CDR-L3 comprising SEQ ID NO: 8. The Fab comprising the first antigen-binding portion binding HVEM is linked to a scFv (clone 52SR4), preferably via a peptide linker to the C-terminal of the CH1 domain of the Fab fragment and the VL domain of the scFv. The scFv may itself also contains a peptide linker located in between its VL and VH domains.

Fab-Y also comprises a Fab fragment which comprises the second antigen-binding portion binding CD9 which comprises a second heavy chain variable region and a second light chain variable region and wherein the second heavy chain variable region comprises a CDR-H1 comprising SEQ ID NO: 9, a CDR-H2 comprising SEQ ID NO: 10 and a CDR-H3 comprising SEQ ID NO: 11; and the second light chain variable region comprises a CDR-L1 comprising SEQ ID NO: 12, a CDR-L2 comprising SEQ ID NO: 13 and a CDR-L3 comprising SEQ ID NO: 14. The Fab comprising the second antigen-binding portion binding CD9 is linked to a peptide GCN4 (clone 7P14P), preferably via a peptide linker to the CH1 domain of the Fab fragment.

The scFv of Fab-X is specific for and complementary to the peptide GCN4 of Fab-Y. As a result, when the Fab-X and the Fab-Y are brought into contact with each other, a non-covalent binding interaction between the scFv and GCN4 peptide occurs, thereby physically retaining the two antigen-binding portions in the form of a complex resulting in an antibody comprising two antigen-binding portions on the same molecule (Figure 1).

In another embodiment, the Fab-X comprises the first antigen-binding portion binding HVEM which comprises a first heavy chain variable region comprising SEQ ID NO: 15 and the first light chain variable region comprising SEQ ID NO: 17; and Fab-Y comprises the second antigen-binding portion binding CD9 which comprises the second heavy chain variable region comprising SEQ ID NO: 19 and second light chain variable region comprising SEQ ID NO: 21.

Binding specificities may be exchanged between Fab-X and Fab-Y, i.e. in one embodiment Fab-X may comprise the antigen-binding portion binding to HVEM and Fab-Y the antigen-binding portion binding to CD9; in another embodiment Fab-X may comprise the antigen-binding portion binding to CD9 and Fab-Y the antigen-binding portion binding to HVEM.

The antibody of the present invention may be comprised in a pharmaceutical composition along with one or more pharmaceutically acceptable excipients. By pharmaceutical composition is intended a composition for both therapeutic and diagnostic use. In another aspect, the present invention provides for a pharmaceutical composition comprising an antibody, preferably a monoclonal antibody, comprising a first antigen-binding portion binding HVEM and a second antigen-binding portion binding CD9, wherein the antibody is preferably humanised and wherein the composition comprises one or more pharmaceutically acceptable excipients.

Pharmaceutically acceptable excipients in therapeutic compositions may additionally contain liquids such as water, saline, glycerol and ethanol. Additionally, auxiliary substances, such as wetting or emulsifying agents or pH buffering substances, may be present in such compositions. Such excipients enable the pharmaceutical compositions to be formulated as tablets, pills, dragees, capsules, liquids, gels, syrups, slurries and suspensions, for ingestion by the patient.

The antibody of the present invention and the pharmaceutical composition comprising such antibody may be used in therapy.

Therefore, in another aspect, the present invention provides for an antibody, preferably a monoclonal antibody, or a pharmaceutical composition comprising the antibody, and one or more pharmaceutically acceptable excipients, wherein the antibody comprises a first antigen-binding portion binding HVEM and a second antigen-binding portion binding CD9, wherein the antibody is preferably humanised and is for use in therapy.

In one embodiment of this further aspect, the antibody or composition comprising such antibody for use in therapy is an antibody comprising a first antigen-binding portion binding HVEM and a second antigen-binding portion binding an extracellular domain region of human CD9, wherein the extracellular domain region of CD9 is preferably loop 2 of CD9, and wherein more preferably the second antigen-binding portion binds within amino acids 112 to 195 of SEQ ID NO: 2, wherein the first and the second antigen-binding portions are located on the same antibody, i.e. they are part of the same polypeptide chain, associate via one or more non-covalent and/or covalent associations or linked so as to form one single molecule.

In another aspect, the present invention provides for an antibody, preferably a monoclonal antibody, or a pharmaceutical composition comprising the antibody, and one or more pharmaceutically acceptable excipients, wherein the antibody comprises a first antigen-binding portion binding HVEM and a second antigen-binding portion binding CD9, wherein the antibody is preferably humanised and is for use in the treatment of cancer and/or an infectious disease.

In another embodiment, the antibody or composition comprising such antibody for use in the treatment of cancer and/or an infectious disease is an antibody comprising a first antigen-binding portion binding HVEM and a second antigen-binding portion binding an extracellular domain region of human CD9, wherein the extracellular domain region of CD9 is preferably loop 2 of CD9, and wherein more preferably the second antigen-binding portion binds within amino acids 112 to 195 of SEQ ID NO: 2, wherein the first and the second antigen-binding portions are located on the same antibody, i.e. they are part of the same polypeptide chain, associate via one or more non-covalent and/or covalent associations or linked so as to form one single molecule.

In yet another aspect, the present invention provides for a method for treating a subject afflicted with cancer and/or an infectious disease, comprising administering to the subject a pharmaceutically effective amount of an antibody, preferably a monoclonal antibody, or a pharmaceutical composition comprising the antibody, and one or more pharmaceutically acceptable excipients, wherein the antibody comprises a first antigen-binding portion binding HVEM and a second antigen-binding portion binding CD9, wherein the antibody is preferably humanised.

The subjects to be treated is preferably a human subject. In one embodiment, there is provided for a method for treating a human subject afflicted with cancer and/or an infectious disease, comprising administering to the subject a pharmaceutically effective amount of an antibody, preferably a monoclonal antibody, or a pharmaceutical composition comprising the antibody and one or more pharmaceutically acceptable excipients, wherein the antibody comprises a first antigen-binding portion binding HVEM and a second antigen-binding portion binding CD9, wherein the antibody is preferably humanised.

In yet another embodiment, the method for treating a human subject afflicted with cancer and/or an infectious disease comprises administering an antibody or composition comprising such antibody comprising a first antigen-binding portion binding HVEM and a second antigen-binding portion binding an extracellular domain region of human CD9, wherein the extracellular domain region of CD9 is preferably loop 2 of CD9, and wherein more preferably the second antigen-binding portion binds within amino acids 112 to 195 of SEQ ID NO: 2, wherein the first and the second antigen-binding portions are located on the same antibody, i.e. they are part of the same polypeptide chain, associate via one or more non-covalent and/or covalent associations or linked so as to form one single molecule.

In another aspect of the present invention, there is provided the use of an antibody, preferably a monoclonal antibody, or a pharmaceutical composition comprising the antibody, and one or more pharmaceutically acceptable excipients, wherein the antibody comprises a first antigen-binding portion binding HVEM and a second antigen-binding portion binding CD9, wherein the antibody is preferably humanised, in the manufacture of a medicament for treating cancer and/or an infectious disease.

Example of cancers that may be treated using the antibody, or pharmaceutical composition comprising such antibody, include but are not limited to, Acute Lymphoblastic Leukaemia, Acute Myeloid Leukaemia, Adrenocortical Carcinoma, AIDS-Related Cancers Kaposi Sarcoma (Soft Tissue Sarcoma), AIDS-Related Lymphoma, Primary CNS Lymphoma, Anal Cancer, Appendix Cancer Astrocytomas, Atypical Teratoid/Rhabdoid Tumour, Brain Cancer, Basal Cell Carcinoma of the Skin, Bile Duct Cancer, Bladder Cancer, Bone Cancer (includes Ewing Sarcoma and Osteosarcoma and Malignant Fibrous Histiocytoma), Breast Cancer, Bronchial Tumours, Burkitt Lymphoma, Carcinoid Tumour, Cardiac (Heart) Tumours, Embryonal Tumours, Germ Cell Tumour, Primary CNS Lymphoma, Cervical Cancer, Cholangiocarcinoma, Chordoma, Chronic Lymphocytic Leukaemia, Chronic Myelogenous Leukaemia, Chronic Myeloproliferative Neoplasms, Colorectal Cancer, Craniopharyngioma, Cutaneous T-Cell Lymphoma, Ductal Carcinoma In Situ, Endometrial Cancer (Uterine Cancer), Ependymoma, Oesophageal Cancer, Esthesioneuroblastoma (Head and Neck Cancer), Ewing Sarcoma (Bone Cancer), Extracranial Germ Cell Tumour, Extragonadal Germ Cell Tumour, Eye Cancer, Intraocular Melanoma, Retinoblastoma, Fallopian Tube Cancer, Fibrous Histiocytoma of Bone, Malignant, and Osteosarcoma, Gallbladder Cancer, Gastric (Stomach) Cancer, Gastrointestinal Carcinoid Tumour, Gastrointestinal Stromal Tumours (Soft Tissue Sarcoma), Ovarian Germ Cell Tumours, Testicular Cancer, Gestational Trophoblastic Disease, Hairy Cell Leukaemia, Head and Neck Cancer, Hepatocellular (Liver) Cancer, Histiocytosis, Langerhans Cell Hodgkin Lymphoma, Hypopharyngeal Cancer, Intraocular Melanoma, Isle T-cell Tumours, Pancreatic Neuroendocrine Tumours, Kaposi Sarcoma (Soft Tissue Sarcoma), Kidney (Renal Cell) Cancer, Langerhans Cell Histiocytosis, Laryngeal Cancer, Leukaemia, Lip and Oral Cavity Cancer, Liver Cancer, Lung Cancer (Non-Small Cell and Small Cell), Male Breast Cancer, Malignant Fibrous Histiocytoma of Bone and Osteosarcoma, Melanoma, Intraocular (Eye) Childhood Intraocular, Merkel Cell Carcinoma, Mesothelioma, Metastatic Cancer, Metastatic Squamous Neck Cancer with Occult Primary, Midline Tract Carcinoma With NUT Gene Changes, Mouth Cancer, Multiple Endocrine Neoplasia Syndromes, Multiple Myeloma/Plasma Cell Neoplasms, Mycosis Fungoides (Lymphoma), Myelodysplastic Syndromes, Myelodysplastic/Myeloproliferative Neoplasms, Myelogenous Leukaemia, Chronic, Myeloid Leukaemia, Acute, Myeloproliferative Neoplasms, Nasal Cavity and Paranasal Sinus Cancer, Nasopharyngeal Cancer, Neuroblastoma, Non-Hodgkin Lymphoma, Non-Small Cell Lung Cancer, Oral Cancer, Lip and Oral Cavity Cancer and Oropharyngeal Cancer, Osteosarcoma and Malignant Fibrous Histiocytoma of Bone, Ovarian Cancer, Pancreatic Cancer, Pancreatic Neuroendocrine Tumours (Isle T-cell Tumours), Papillomatosis, Paraganglioma, Paranasal Sinus and Nasal Cavity Cancer, Parathyroid Cancer, Penile Cancer, Pharyngeal Cancer, Pheochromocytoma, Pituitary Tumour, Plasma Cell Neoplasm/Multiple Myeloma, Pleuropulmonary Blastoma, Pregnancy and Breast Cancer, Primary Central Nervous System (CNS) Lymphoma, Primary Peritoneal Cancer, Prostate Cancer, Rectal Cancer, Recurrent Cancer, Renal Cell (Kidney) Cancer, Retinoblastoma, Rhabdomyosarcoma, Salivary Gland Cancer, Sarcoma Childhood Rhabdomyosarcoma, Childhood Vascular Tumours, Ewing Sarcoma, Kaposi Sarcoma, Osteosarcoma, Soft Tissue Sarcoma, Uterine Sarcoma, Sézary Syndrome, Skin Cancer, Small Intestine Cancer, Squamous Cell Carcinoma of the Skin, Squamous Neck Cancer with Occult Primary, Stomach (Gastric) Cancer, Cutaneous T-Cell Lymphoma, Testicular Cancer, Throat Cancer, Nasopharyngeal Cancer, Oropharyngeal Cancer, Hypopharyngeal Cancer, Thymoma and Thymic Carcinoma, Thyroid Cancer, Transitional Cell Cancer of the Renal Pelvis and Ureter, Urethral Cancer, Uterine Cancer, Endometrial Uterine Sarcoma, Vaginal Cancer, Vascular Tumours, Vulvar Cancer and Wilms Tumour, and any combinations of said cancers. The present invention is also applicable to treatment of metastatic cancers.

The antibody according to the present invention, or the pharmaceutical composition comprising such antibody, may be administered concomitantly or sequentially to one or more additional cancer therapies. By cancer therapies is intended drug-based therapies as well as other type of cancer therapies such as radiotherapies.

The invention will now be further described by way of examples with references to embodiments illustrated in the accompanying drawings

### EXAMPLES

### Example 1: T-cell activation primary screen

The activation status of T-cells can be assessed through their expression of cell surface markers and secreted cytokines which play important roles in cellular function. Activated T-cells for example upregulate CD25, CD71 and CD137 on their surface. T-cells in the tumour microenvironment are often maintained in a suppressed state and express only low levels of these proteins. Agents that overcome this suppression and induce T-cell activation and proliferation have tremendous therapeutic potential as they may unleash effective anti-tumour T-cell responses and promote cancer elimination.

To identify novel modulators of T-cell activation, a large screen was undertaken whereby 49 antigen specificities were combined to generate a grid of bispecific antibodies with a theoretical size of 1,176 possible bispecific combinations. The specificities were selected from the literature as being expressed on T-cells or expressed on other cell types involved in interactions with T-cells. Of this potential grid, 969 novel antigen bispecific constructs were tested, covering 82.4% of the possible combinations. Between 1 and 4 different antibodies were tested for each specificity, and all were tested on peripheral blood mononuclear cells (PBMC) from donors in combination with a negative control arm to identify the effect of the monovalent forms of the construct.

PBMC represent the major leukocyte classes involved in both innate and adaptive immunity, apart from granulocytes. PBMC comprise a heterogenous population of cells which when manipulated *in vitro* provide a relatively more relevant physiological environment compared to isolated component cell types such as T-cell types such as T-cells and monocytes, that are no longer capable of responding to paracrine and autocrine signals provided by other cells. As such identification of molecules modulating specific subsets of cells within the wider PBMC population, have increased translational potential to more complex biological systems, ultimately increasing success rates for modulating immune cell interactions in disease.

Each bispecific combination was tested on two PBMC donors. The negative control arm is a Fab from an antibody raised to an antigen not expressed on PBMC.

Fusion proteins were prepared according to the disclosure of WO2015/181282, WO2016/009030, WO2016/009029, WO2017/093402, WO2017/093404 and WO2017/093406. The first fusion protein (A-X) includes a Fab fragment (A of the A-X) with specificity to one antigen, which is linked to X, a scFv (clone 52SR4) via a peptide linker to the C-terminal of the CH1 domain of the Fab fragment and the VL domain of the scFv. The scFv itself also contains a peptide linker located in between its VL and VH domains.

The second fusion protein (B-Y) includes a Fab fragment (B of the B-Y) with specificity to another antigen. However, in comparison to the first protein, the Fab fragment B is attached to Y, a peptide GCN4 (clone 7P14P) via a peptide linker to the CH1 domain of the Fab fragment.

The scFv, X, is specific for and complementary to the peptide GCN4, Y. As a result, when the two fusion proteins are brought into contact with each other, a non-covalent binding interaction between the scFv and GCN4 peptide occurs, thereby physically retaining the two fusion proteins in the form of a complex mimicking an antibody comprising two different antigen-binding portions on the same molecule (Figure 2).

Purified Fab-X and Fab-Y with varying specificities were incubated together for 60 minutes (in a 37°C/5% CO₂ environment) at an equimolar concentration. The final molarity of each tested complex was 100 nM. In 384-well tissue culture plates, 1.0E+05 PBMC were added to wells, to which were added pre-formed Fab-X/Fab-Y bispecific antibodies. Following addition of the bispecific antibodies, the cells were incubated for 48 hours at 37°C/5% CO₂, with or without 1 µg/mL (final concentration) Staphylococcal Enterotoxin-B (SEB) superantigen. After 48 hours the plates were centrifuged at 500 × g for 5 minutes at 4°C. Cell culture conditioned media was transferred from the cell pellets to fresh plates and frozen at -80°C. Cells were washed with 60 µL FACS buffer two times by centrifugation, followed by resuspension of the pellets by shaking the plates at 2200 rpm for 30 seconds. The cells were stained with a panel of fluorescently labelled antibodies as listed in the Table 2 and incubated at 4°C in the dark for 1 hour.

**Table 2**

| **Epitope** | **Fluorophore** | **Clone** | **Source** | **Dilution** |
|---|---|---|---|---|
| CD56 | APC | HCD56 | BioLegend^{®} | 40 |
| CD14 | PE/Cy7 | M5E2 | BD Pharmagen^{®} | 80 |
| CD8 | BV650 | RPA-T8 | BioLegend^{®} | 80 |
| CD4 | BV605 | RPA-T4 | BD Horizon^{®} | 40 |
| CD19 | PE-Cy5 | HIB19 | BD Pharmagen^{®} | 20 |
| CD45RO | BV786 | UCHL1 | BD Horizon^{®} | 40 |
| CD71 | FITC | M-A712 | BD Pharmagen^{®} | 20 |
| CD25 | BV510 | M-A251 | BD Horizon^{®} | 40 |
| CD137 | PE | 4B4-1 | BD Pharmagen^{®} | 20 |
| Live/Dead | Near IR | | Thermo Scientific^{®} | 1000 |

After this time, cells were washed twice with FACS buffer, before fixing with 2% paraformaldehyde (BD CellFix^{™}, diluted in dH₂O) and stored overnight at 4°C. The plates were centrifuged at 500 × g for 5 minutes, the fixation buffer aspirated to waste and the cells resuspended in a residual volume of 10 µL for acquisition on the iQue^{®} Screener Plus (IntelliCyt^{®}). The data analysis software package ForeCyt^{™} (IntelliCyt^{®}) was used to gate on CD14+ monocytes, CD56+ NK cells, CD19+ B-cells, CD4+ and CD8+ memory and naive T-cells. For each cell population the cellular expression of CD25 and CD71 was measured as reported median fluorescent intensity values. The data were then used to calculate the log2 fold changes of expression relative to control well values.

The resulting flow cytometry data were analysed by identification of the CD4+ and CD8+ memory and naive T-cells and well as the B cells, NK cells and monocytes. The MFI values of the 3 activation markers CD71, CD137 and CD25 were then determined and the log2 fold changes calculated relative to the control wells for each cell population. These results were deposited into the visualisation software Spotfire^{®}, where it was possible to identify antigen pairs capable of inducing an increase in T-cell activation.

When considering antigen pairs capable of upregulating the activation markers CD25, CD137 and CD71 on T-cells in the presence of an SEB stimulation, but not in unstimulated conditions, the HVEM-CD9 bispecific pair was identified.

The HVEM-CD9 bispecific antibody was therefore taken into subsequent assays to show that its effect was reproducible across a larger number of donors.

### Example 2: HVEM-CD9 follow up assay

To confirm the effect of HVEM-CD9 on stimulation of T-cells, a further 4 PBMC donors were assayed in SEB-stimulated and unstimulated conditions.

A grid of fusion proteins Fab-X and Fab-Y were created by diluting equimolar (1 µM) quantities of Fab-X (Fab-scFv) and Fab-Y (Fab-peptide) with specificity for HVEM and CD9 in TexMACS^{™} media (Miltenyi Biotec^{®}) containing 100 U/mL penicillin/100 µg/mL streptomycin. Mixtures of equimolar (1 µM) Fab-Y proteins were also generated in the same manner. The Fab-X and Fab-Y fusion proteins were incubated together for 1 hour (in a 37°C/5%CO₂ environment), at a final concentration of 500 nM. Negative control wells containing TexMACS^{™} media only were also generated alongside the Fab-X and Fab-Y wells.

During this time, cryopreserved human PBMCs isolated from platelet leukapheresis cones were thawed and washed in TexMACS^{™} media and resuspended at 3.33 × 10⁶ cells/mL. The PBMCs were then seeded into 384 well flat bottom tissue culture plates (Greiner Bio-one^{®}) at 30 µl/well (1×10⁵ PBMCs).

A total of 10 µl of Fab-X and Fab-Y complexes were transferred to the plates containing 30 µl PBMCs. The PBMCs were then either left unstimulated by the addition of 10 µl of TexMACS^{™} media, or stimulated with 10µl of SEB (1 µg/mL final concentration). This resulted in a final assay concentration of Fab-X and Fab-Y complexes of 100 nM. The plates were then returned to a 37°C/5%CO₂ environment for 48 hours.

After 48 hours the plates were centrifuged at 500 × g for 5 minutes at 4°C. Conditioned medium was transferred from the cell pellets to fresh plates and frozen at -80°C. Cells were washed with 60 µL FACS buffer two times by centrifugation, followed by resuspension of the pellets by shaking the plates at 2200 rpm for 30 seconds. The cells were stained with a panel of fluorescently labelled antibodies as listed in Table 2 and incubated at 4°C in the dark for 1 hour. After this time, cells were washed twice with FACS buffer, before fixing with 2% paraformaldehyde (BD CellFix^{™}, diluted in dH₂O) and stored overnight at 4°C. The plates were centrifuged at 500 × g for 5 minutes, the fixation buffer aspirated to waste and the cells resuspended in a residual volume of 10 µL for acquisition on the iQue^{®} Screener Plus (IntelliCyt^{®}).

The data analysis software package ForeCyt^{™} (IntelliCyt^{®}) was used to gate on CD14+ monocytes, CD56+ NK cells, CD19+ B-cells, CD4+ and CD8+ memory and naive T-cells. For each cell population the cellular expression of CD25, CD71 and CD137 was measured as reported median fluorescent intensity values. The data were then used to calculate the log2 fold changes of expression relative to control well values.

Twenty-Four Fab-X/Fab-Y bispecific antibodies showed increased expression of the activation marker CD25 on CD4+ (Figure 3) and CD8+ (Figure 4) T-cells with SEB stimulation, while the control constructs did not lead to this increase. The bivalents (i.e. formed by a fusion where both Fabs in the Fab-X and Fab-Y are specific for CD9 or HVEM as the case may be) and monovalent antibodies for CD9 or HVEM (i.e. formed by a fusion where the Fab is specific for CD9 or HVEM but the other component Fab is a negative control) did not lead to a similar increase in the activation marker fluorescence intensity on either cell populations, suggesting that a monospecific antibody binding CD9 alone or HVEM alone, whether in a monovalent or bivalent fashion, is unable to stimulate activation in the absence of the binding to the other antigen. The small increases in CD25 expression detected by the HVEM bivalent and monovalent controls in CD4+ T-cells, however were only small increases, which could only be greatly enhanced by the co-targeting of CD9. Furthermore, the mixture of an antibody binding solely to HVEM and an antibody binding solely to CD9 (Fab-Y mixture) also had no enhanced stimulatory effect on the T-cell populations compared to the HVEM controls, implying the requirement for the antigen-binding portions binding HVEM and CD9 to be on the same chain, associated via non-covalent associations or linked for the stimulatory function to occur.

When studied in unstimulated conditions the HVEM/CD9 bispecific antibodies did not lead to any increases in activation marker fluorescence intensity in either CD4+ cells (Figure 5) or CD8+ cells (Figure 6). This confirms that the binding of both HVEM and CD9 does not lead to the unwanted activation of resting T-cells.

Furthermore, it was possible to measure the level of CD25 expression on naive versus memory T-cell populations by the separation of the CD45RO-expressing memory cells from the CD45RO non-expressing naive T-cell populations. Upon analysis of these sub populations a preference for upregulation of CD25 on the memory CD4+ and CD8+ T-cell populations compared to the naïve populations was identified (Figures 7-10). This suggests that HVEM-CD9 bispecific antibodies could preferentially target the memory T-cell populations, enhancing their therapeutic potential.

The levels of expression on CD4+ and CD8+ memory T-cells of CD71 (Figures 11 and 12, respectively) and CD137 (Figures 13 and 14, respectively) was also investigated. The HVEM/CD9 bispecific antibodies showed an effect resulting in increased levels of expression of CD71 and CD137 markers, irrespective of the orientation of the Fab-X/Fab-Y bispecific antibody.

The bivalents (i.e. formed by a fusion where both Fab in the Fab-X and Fab-Y are specific for CD9 or HVEM as the case may be) and monovalent antibodies for CD9 or HVEM (i.e. formed by a fusion where the Fab is specific for CD9 or HVEM but the other component Fab is a negative control) did not lead to a similar increase in the activation marker fluorescence intensity on either cell populations, suggesting that a monospecific antibody binding CD9 alone or HVEM alone, whether in a monovalent or bivalent fashion, is unable to stimulate activation in the absence of the binding to the other antigen.

### Example 3: Effect of a HVEM-CD9 bispecific antibody on T-cell proliferation

The effect of an anti-HVEM-CD9 bispecific antibody on proliferation of CD4+ and CD8+ T-cells was assessed in 5 PBMC donors. Mixtures of fusion proteins Fab-X and Fab-Y were created by diluting equimolar (1 µM) quantities of Fab-X and Fab-Y with specificity for HVEM and CD9 in DMEM (Thermo Fisher) containing 10% foetal bovine serum (FBS) and 100 U/mL penicillin/100 µg/mL streptomycin. Mixtures of equimolar (1 µM) Fab-Y proteins were also generated in the same manner. The Fab-X and Fab-Y fusion proteins were incubated together for 1 hour (in a 37°C/5%CO₂ environment) to generate bispecific antibodies.

During this time, cryopreserved human PBMCs isolated from platelet leukapheresis cones were thawed, washed in DMEM media and resuspended at approximately 2 ×10⁶ cells/mL in PBS. CellTrace^{™} Violet (CTV; ThermoFisher) was then added to a final concentration of 1 µM (2 µl 5 mM CTV in DMSO added to 10mL cells), incubated in the dark at room temperature for 20 minutes, the cells washed twice with DMEM and resuspended in media to a final concentration of 1 ×10⁶ cells/mL.

Fab-X/Fab-Y bispecific antibodies were diluted to 400 nM concentration in DMEM and 50 µl transferred in triplicate to wells of a 96 well U bottom tissue culture plate. Anti-CD3 (clone UCHT-1), 50µl of a 200ng/mL solution in DMEM, was then added. To 3 wells 100 µl DMEM media alone was added as a negative proliferation control. Finally, 100 µl of CTV labelled PBMC were added to each well. This resulted in a final assay concentration of Fab-X/Fab-Y bispecific antibodies of 100 nM, 50 ng/mL anti-CD3 and 1 × 10⁵ cells/well. The plates were then returned to a 37°C/5%CO₂ environment for 96 hours.

After 96 hours the plates were centrifuged at 300 × g for 5 minutes. Cell culture conditioned media was removed, the cells were washed twice with FACS buffer (2%FBS in PBS) and then resuspended in 50 µl FACS buffer containing fluorescently labelled antibodies, as listed in Table 3. The cells were incubated at room temperature in the dark for 20 minutes, washed twice with FACS buffer, resuspended in 100 µl/per well FACS buffer and analysed by flow cytometry (BD FACS Canto II^{™}). Total events from 50 µl (50% volume) of each well were collected.

The data analysis software package FlowJo^{®} was used to gate on CD8+ and CD4+ cells. Cell proliferation was assessed by enumerating cells with reduced CTV staining relative to unstimulated cells. The data are presented as the mean ± SEM for triplicate wells.

**Table 3**

| **Epitope** | **Fluorophore** | **Clone** | **Source** | **Dilution** |
|---|---|---|---|---|
| CD8 | PE | RPA-T8 | BD | 100 |
| CD4 | APC | RPA-T4 | BioLegend^{®} | 20 |

As shown in Figure 15, proliferation of CD8+ and CD4+ T-cells in the presence of anti-CD3 (50 ng/mL) stimulation was statistically significantly higher when human PBMC were treated with the HVEM/CD9 bispecific antibody versus the negative control (no bispecific antibody) p<0.01 (Mann-Whitney test)

### Example 4: Analysis of anti-CD9 antibody binding by biolayer interferometry

CD9 contains two extracellular loops: a short extracellular loop (loop 1: 34-55 in SEQ ID NO:2) and a long extracellular loop (loop 2: 112-195 in SEQ ID NO:2). We used biolayer interferometry to assess binding of our panel of CD9 Fab-Y antibodies (selected for their ability to bind full-length cell-expressed CD9) to the long extracellular loop 2 peptide, using the Octet^{®} RED384 System (FortéBio) with Anti-hlgG Fc Capture (AHC) Biosensors (FortéBio) at room temperature. Firstly, an array of 8 sensors was dipped in kinetics buffer (PBS 0.1%, BSA 0.02%, Tween 20) for 120 seconds to provide a baseline signal. Sensors were then moved to wells containing 200 µl of recombinant human CD9 long extracellular loop 2-human Fc fusion protein (10015-CD, R&D Systems^{®}) at 2 µg/mL in kinetics buffer to immobilize the protein to the biosensor (100 seconds), followed by a second baseline step (180 seconds) in kinetics buffer to equilibrate the biosensors now coated with the CD9 long extracellular loop 2-human Fc fusion protein. No detachment of the peptide was observed during this step. Sensors were then dipped in wells containing one of the anti-CD9 Fab (10 µg/mL) to evaluate association of each antibody to CD9-loop2 (120 seconds), followed by dissociation in kinetics buffer (600 seconds). Anti-CD137 Fab-Y (11175) was used as negative control during the antibody association step. A new set of 8 biosensors was used to repeat this process for each of the anti-CD9 antibodies (7270, 7271, 7272, 7485, 7486, 7489, 7491).

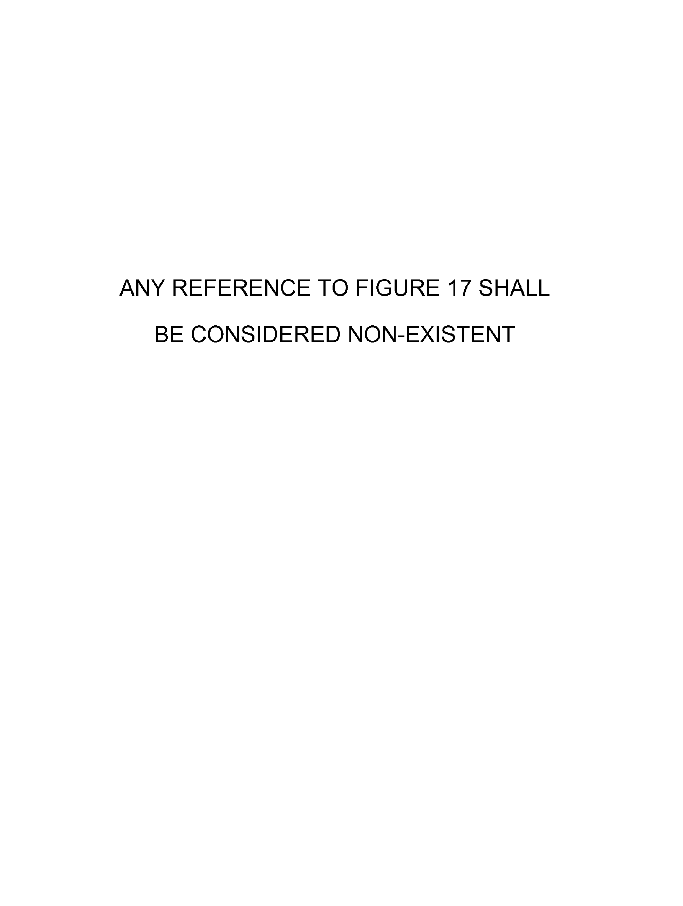

As shown in Table 4, all the anti-CD9 antibodies tested bind to the long extracellular loop 2 of CD9 and all are functional when combined with anti-HVEM antibodies 7660 and 7817. A positive functional response is considered to be the capacity to increase CD25 expression on T cells greater than 0.5 log2 fold change MFI in 2 donors when added as a Fab-K_{D}-Fab with anti- HVEM antibodies as shown in generated as in example 2 and shown in Figure 17.

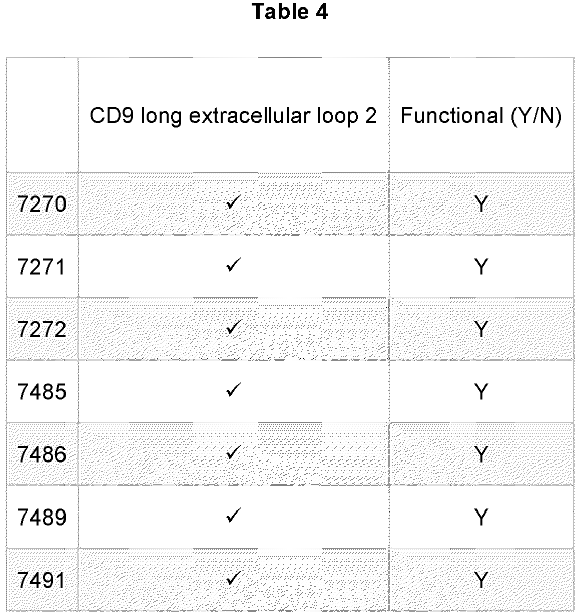

## Claims

1. An antibody which comprises a first antigen-binding portion binding HVEM and a second antigen-binding portion binding CD9.

2. The antibody according to claim 1, wherein each of the antigen-binding portions is a monoclonal antigen-binding portion.

3. The antibody according to claim 1 or claim 2, wherein each of the antigen-binding portions is independently selected from a Fab, a Fab', a scFv or a VHH.

4. The antibody according to claim 1 or claim 2, wherein the antigen-binding portions are the antigen-binding portions of an IgG.

5. The antibody according to any one of the preceding claims wherein the antibody is chimeric, human or humanised, preferably the antibody is humanised.

6. The antibody according to any one of the preceding claims wherein the antibody comprises a heavy chain constant region selected from an IgG1, an IgG2, IgG3 or an IgG4
isotype.

7. The antibody according to anyone of the preceding claims, wherein the antibody further comprises at least an additional antigen-binding portion; wherein preferably the additional antigen-binding portion is capable of increasing the half-life of the antibody.

8. The antibody according to claim 7, wherein the additional antigen-binding portion binds albumin, preferably human serum albumin.

9. The antibody according to any one of the preceding claims wherein the second antigen binding portion binds CD9 in CD9 loop 2, wherein preferably the second antigen-binding portion binds within amino acids 112 to 195 of SEQ ID NO: 2.

10. The antibody according to any one of the preceding claims wherein the first antigen-binding portion binding HVEM comprises a first heavy chain variable region and a first light chain variable region and the second antigen-binding portion binding CD9 comprises a second heavy chain variable region and a second light chain variable region and wherein:
a. The first heavy chain variable region comprises a CDR-H1 comprising SEQ ID NO: 3, a CDR-H2 comprising SEQ ID NO: 4 and a CDR-H3 comprising SEQ ID NO: 5; and
b. The first light chain variable region comprises a CDR-L1 comprising SEQ ID NO: 6, a CDR-L2 comprising SEQ ID NO: 7 and a CDR-L3 comprising SEQ ID NO: 8; and
c. The second heavy chain variable region comprises a CDR-H1 comprising SEQ ID NO: 9, a CDR-H2 comprising SEQ ID NO: 10 and a CDR-H3 comprising SEQ ID NO: 11; and
d. The second light chain variable region comprises a CDR-L1 comprising SEQ ID NO: 12, a CDR-L2 comprising SEQ ID NO: 13 and a CDR-L3 comprising SEQ ID NO: 14;
or
e. The first heavy chain variable region comprises SEQ ID NO: 15 and the first light chain variable region comprises SEQ ID NO: 17; and the second heavy chain variable region comprises SEQ ID NO: 19 and second light chain variable region comprises SEQ ID NO: 21;
or
f. The first heavy chain variable region is encoded by a nucleotide sequence comprising SEQ ID NO: 16 and the first light chain variable region is encoded by a nucleotide sequence comprising SEQ ID NO: 18; and the second heavy chain variable region is encoded by a nucleotide sequence comprising SEQ ID NO: 20 and second light chain variable region is encoded by a nucleotide sequence comprising SEQ ID NO: 22.

11. A pharmaceutical composition comprising the antibody according to any one of the preceding claims and one or more pharmaceutically acceptable excipients.

12. The antibody according to any one of claims 1 to 10 or the pharmaceutical composition according to claim 11 for use in therapy.

13. The antibody according to any one of claims 1 to 10 or the pharmaceutical composition according to claim 11 for use in the treatment of cancer and/or an infectious disease.

14. The antibody for use according to claim 13, wherein the antibody or the composition are administered concomitantly or sequentially to one or more additional cancer therapies.

## Patentansprüche

1. Antikörper, der einen ersten antigenbindenden Teil, der HVEM bindet, und einen zweiten antigenbindenden Teil, der CD9 bindet, umfasst.

2. Antikörper nach Anspruch 1, wobei es sich bei den antigenbindenden Teilen jeweils um einen monoklonalen antigenbindenden Teil handelt.

3. Antikörper nach Anspruch 1 oder Anspruch 2, wobei die antigenbindenden Teile jeweils unabhängig aus einem Fab, einem Fab', einem scFv oder einer VHH ausgewählt sind.

4. Antikörper nach Anspruch 1 oder Anspruch 2, wobei es sich bei den antigenbindenden Teilen um die antigenbindenden Teile eines IgG handelt.

5. Antikörper nach einem der vorhergehenden Ansprüche, wobei es sich um einen chimären, menschlichen oder humanisierten Antikörper, bevorzugt um einen humanisierten Antikörper handelt.

6. Antikörper nach einem der vorhergehenden Ansprüche, wobei der Antikörper eine aus einem IgG1-, einem IgG2-, IgG3- oder einem IgG4-Isotyp ausgewählte konstante Region der schweren Kette umfasst.

7. Antikörper nach einem der vorhergehenden Ansprüche, wobei der Antikörper ferner wenigstens einen zusätzlichen antigenbindenden Teil umfasst; wobei der zusätzliche antigenbindende Teil bevorzugt die Halbwertszeit des Antikörpers erhöhen kann.

8. Antikörper nach Anspruch 7, wobei der zusätzliche antigenbindende Teil Albumin, bevorzugt Humanserumalbumin bindet.

9. Antikörper nach einem der vorhergehenden Ansprüche, wobei der zweite antigenbindende Teil CD9 in CD9-Schleife 2 bindet, wobei der zweite antigenbindende Teil innerhalb Aminosäuren 112 bis 195 von SEQ ID NO: 2 bindet.

10. Antikörper nach einem der vorhergehenden Ansprüche, wobei der HVEM bindende erste antigenbindende Teil eine erste variable Region der schweren Kette und eine erste variable Region der leichten Kette umfasst und der CD9 bindende zweite antigenbindende Teil eine zweite variable Region der schweren Kette und eine zweite variable Region der leichten Kette umfasst und wobei:
a. die erste variable Region der schweren Kette eine CDR-H1, die SEQ ID NO: 3 umfasst, eine CDR-H2, die SEQ ID NO: 4 umfasst, und eine CDR-H3, die SEQ ID NO: 5 umfasst, umfasst; und
b. die erste variable Region der leichten Kette eine CDR-L1, die SEQ ID NO: 6 umfasst, eine CDR-L2, die SEQ ID NO: 7 umfasst, und eine CDR-L3, die SEQ ID NO: 8 umfasst, umfasst; und
c. die zweite variable Region der schweren Kette eine CDR-H1, die SEQ ID NO: 9 umfasst, eine CDR-H2, die SEQ ID NO: 10 umfasst, und eine CDR-H3, die SEQ ID NO: 11 umfasst, umfasst; und
d. die zweite variable Region der leichten Kette eine CDR-L1, die SEQ ID NO: 12 umfasst, eine CDR-L2, die SEQ ID NO: 13 umfasst, und eine CDR-L3, die SEQ ID NO: 14 umfasst, umfasst;
oder
e. die erste variable Region der schweren Kette SEQ ID NO: 15 und die erste variable Region der leichten Kette SEQ ID NO: 17 umfasst; und die zweite variable Region der schweren Kette SEQ ID NO: 19 und die zweite variable Region der leichten Kette SEQ ID NO: 21 umfasst;
oder
f. die erste variable Region der schweren Kette durch eine Nukleotidsequenz, die SEQ ID NO: 16 umfasst, und die erste variable Region der leichten Kette durch eine Nukleotidsequenz, die SEQ ID NO: 18 umfasst, codiert ist; und die zweite variable Region der schweren Kette durch eine Nukleotidsequenz, die SEQ ID NO: 20 umfasst, und die zweite variable Region der leichten Kette durch eine Nukleotidsequenz, die SEQ ID NO: 22 umfasst, codiert ist.

11. Pharmazeutische Zusammensetzung, umfassend den Antikörper nach einem der vorhergehenden Ansprüche und einen oder mehrere pharmazeutisch unbedenkliche Exzipienten.

12. Antikörper nach einem der Ansprüche 1 bis 10 oder pharmazeutische Zusammensetzung nach Anspruch 11 zur therapeutischen Verwendung.

13. Antikörper nach einem der Ansprüche 1 bis 10 oder pharmazeutische Zusammensetzung nach Anspruch 11 zur Verwendung bei der Behandlung von Krebs und/oder einer Infektionskrankheit.

14. Antikörper zur Verwendung nach Anspruch 13, wobei der Antikörper oder die Zusammensetzung gleichzeitig mit einer oder mehreren zusätzlichen Krebstherapien oder darauf folgend verabreicht wird.

## Revendications

1. Anticorps qui comprend une première partie de liaison à un antigène se liant à HVEM et une deuxième partie de liaison à un antigène se liant à CD9.

2. Anticorps selon la revendication 1, chacune des parties de liaison à un antigène étant une partie de liaison à un antigène monoclonal.

3. Procédé selon la revendication 1 ou la revendication 2, chacune des parties de liaison à un antigène étant indépendamment choisie parmi un Fab, un Fab', un scFv ou un VHH.

4. Procédé selon la revendication 1 ou la revendication 2, les parties de liaison à un antigène étant les parties de liaison à un antigène d'une IgG.

5. Anticorps selon l'une quelconque des revendications précédentes, l'anticorps étant chimérique, humain ou humanisé, préférablement l'anticorps étant humanisé.

6. Anticorps selon l'une quelconque des revendications précédentes, l'anticorps comprenant une région constante de chaîne lourde choisie parmi un isotype IgG1, un isotype IgG2, un isotype IgG3 ou un isotype IgG4.

7. Anticorps selon l'une quelconque des revendications précédentes, l'anticorps comprenant en outre au moins une partie de liaison à un antigène supplémentaire ; préférablement la partie de liaison à un antigène supplémentaire étant capable d'augmenter la demi-vie de l'anticorps.

8. Anticorps selon la revendication 7, la partie de liaison à un antigène supplémentaire se liant à une albumine, préférablement une albumine sérique humaine.

9. Anticorps selon l'une quelconque des revendications précédentes, la deuxième partie de liaison à un antigène se liant à CD9 dans la boucle 2 de CD9, préférablement la deuxième partie de liaison à un antigène se liant dans les acides aminés 112 à 195 de la SEQ ID NO: 2.

10. Anticorps selon l'une quelconque des revendications précédentes, la première partie de liaison à un antigène se liant à HVEM comprenant une première région variable de chaîne lourde et une première région variable de la chaîne légère et la deuxième partie de liaison à un antigène se liant à CD9 comprenant une deuxième région variable de chaîne lourde et une deuxième région variable de chaîne légère et :
a. la première région variable de chaîne lourde comprenant une CDR-H1 comprenant la SEQ ID NO: 3, une CDR-H2 comprenant la SEQ ID NO: 4 et une CDR-H3 comprenant la SEQ ID NO: 5 ; et
b. la première région variable de chaîne légère comprenant une CDR-L1 comprenant la SEQ ID NO: 6, une CDR-L2 comprenant la SEQ ID NO: 7 et une CDR-L3 comprenant la SEQ ID NO: 8 ; et
c. la deuxième région variable de chaîne lourde comprenant une CDR-H1 comprenant la SEQ ID NO: 9, une CDR-H2 comprenant la SEQ ID NO: 10 et une CDR-H3 comprenant la SEQ ID NO: 11 ; et
d. la deuxième région variable de chaîne légère comprenant une CDR-L1 comprenant la SEQ ID NO: 12, une CDR-L2 comprenant la SEQ ID NO: 13 et une CDR-L3 comprenant la SEQ ID NO: 14 ;
ou
e. la première région variable de chaîne lourde comprenant la SEQ ID NO: 15 et la première région variable de chaîne légère comprenant la SEQ ID NO: 17 ; et la deuxième région de variable de chaîne lourde comprenant la SEQ ID NO: 19 et la deuxième région variable de chaîne légère comprenant la SEQ ID NO: 21 ;
ou
f. la première région variable de chaîne lourde étant codée par une séquence de nucléotides comprenant la SEQ ID NO: 16 et la première région variable de chaîne légère étant codée par une séquence de nucléotides comprenant la SEQ ID NO: 18 ; et la deuxième région variable de chaîne lourde étant codée par une séquence de nucléotides comprenant la SEQ ID NO: 20 et la deuxième région variable de chaîne légère étant codée par une séquence de nucléotides comprenant la SEQ ID NO: 22.

11. Composition pharmaceutique comprenant l'anticorps selon l'une quelconque des revendications précédentes et un ou plusieurs excipients pharmaceutiquement acceptables.

12. Anticorps selon l'une quelconque des revendications 1 à 10 ou composition pharmaceutique selon la revendication 11 pour une utilisation en thérapie.

13. Anticorps selon l'une quelconque des revendications 1 à 10 ou composition pharmaceutique selon la revendication 11 pour une utilisation dans le traitement d'un cancer et/ou d'une maladie infectieuse.

14. Anticorps pour une utilisation selon la revendication 13, l'anticorps ou la composition étant administré(e) de manière concomitante ou de manière séquentielle par rapport à une ou plusieurs thérapies cancéreuses supplémentaires.
